Europäisches Patentamt

European Patent Office

Office européen des brevets

(19)

(11) **EP 1 110 969 A1**

(12) **EUROPEAN PATENT APPLICATION**
published in accordance with Art. 158(3) EPC

(43) Date of publication:
**27.06.2001 Bulletin 2001/26**

(51) Int Cl.[7]: **C07K 14/54**, C12P 21/02,
C12N 15/24, A61K 38/20

(21) Application number: **98954727.8**

(22) Date of filing: **18.11.1998**

(86) International application number:
**PCT/JP98/05186**

(87) International publication number:
**WO 00/12555 (09.03.2000 Gazette 2000/10)**

(84) Designated Contracting States:
**AT BE CH CY DE DK ES FI FR GB GR IE IT LI LU
MC NL PT SE**

(30) Priority: **01.09.1998 JP 24758898**
**18.11.1998 JP 32791498**

(71) Applicant: **Kabushiki Kaisha Hayashibara
Seibutsu Kagaku Kenkyujo
Okayama-shi, Okayama 700-0907 (JP)**

(72) Inventors:
• **TORIGOE, Kakuji
Kurashiki-shi Okayama 710-0133 (JP)**
• **TANIAI, Madoka
Okayama-shi Okayama 700-0802 (JP)**
• **KURIMOTO, Masashi
Okayama-shi Okayama 700-0011 (JP)**

(74) Representative: **Daniels, Jeffrey Nicholas et al
Page White & Farrer
54 Doughty Street
London WC1N 2LS (GB)**

(54) **INTERLEUKIN 18-BINDING PROTEIN**

(57)     The objects of this invention are to provide a substance which suppresses the physiological activities of IL-18 through binding to IL-18, uses of the substance, and a DNA encoding the substance; this invention attains these objects by providing an IL-18-binding protein comprising a specific amino acid sequence, a DNA encoding this protein, and an IL-18-suppressor as well as agent for susceptive diseases containing as an effective ingredient this IL-18-binding protein.

EP 1 110 969 A1

SEQUENCE LISTING

(1) INFORMATION FOR SEQ ID NO: 1

    (i) SEQUENCE CHARACTERISTICS:

        (A) LENGTH: 164 amino acids

        (B) TYPE: amino acid

        (D) TOPOLOGY: linear

    (Xi) SEQUENCE DESCRIPTION: SEQ ID NO: 1

Thr Pro Val Ser Gln Thr Thr Thr Ala Ala Thr Ala Ser Val Arg Ser
1               5                   10                  15

Thr Lys Asp Pro Cys Pro Ser Gln Pro Pro Val Phe Pro Ala Ala Lys
                20                  25                  30

Gln Cys Pro Ala Leu Glu Val Thr Trp Pro Glu Val Glu Val Pro Leu
                35                  40                  45

Asn Gly Thr Leu Ser Leu Ser Cys Val Ala Cys Ser Arg Phe Pro Asn
            50                  55                  60

Phe Ser Ile Leu Tyr Trp Leu Gly Asn Gly Ser Phe Ile Glu His Leu
65                  70                  75                  80

Pro Gly Arg Leu Trp Glu Gly Ser Thr Ser Arg Glu Arg Gly Ser Thr
                85                  90                  95

Gly Thr Gln Leu Cys Lys Ala Leu Val Leu Glu Gln Leu Thr Pro Ala
                100                 105                 110

Leu His Ser Thr Asn Phe Ser Cys Val Leu Val Asp Pro Glu Gln Val
            115                 120                 125

Val Gln Arg His Val Val Leu Ala Gln Leu Trp Ala Gly Leu Arg Ala
            130                 135                 140

Thr Leu Pro Pro Thr Gln Glu Ala Leu Pro Ser Ser His Ser Ser Pro
145                 150                 155                 160

Gln Gln Gln Gly

.

(2) INFORMATION FOR SEQ ID NO: 2

    (i) SEQUENCE CHARACTERISTICS:

        (A) LENGTH: 165 amino acids

        (B) TYPE: amino acid

        (D) TOPOLOGY: linear

    (Xi) SEQUENCE DESCRIPTION: SEQ ID NO: 2


Thr Ser Ala Pro Gln Thr Thr Ala Thr Val Leu Thr Gly Ser Ser Lys
1               5                       10                      15

Asp Pro Cys Ser Ser Trp Ser Pro Ala Val Pro Thr Lys Gln Tyr Pro
                20                      25                      30

Ala Leu Asp Val Ile Trp Pro Glu Lys Glu Val Pro Leu Asn Gly Thr
                35                      40                      45

Leu Thr Leu Ser Cys Thr Ala Cys Ser Arg Phe Pro Tyr Phe Ser Ile
        50                      55                      60

Leu Tyr Trp Leu Gly Asn Gly Ser Phe Ile Glu His Leu Pro Gly Arg
65                      70                      75                      80

Leu Lys Glu Gly His Thr Ser Arg Glu His Arg Asn Thr Ser Thr Trp
                85                      90                      95

Leu His Arg Ala Leu Val Leu Glu Glu Leu Ser Pro Thr Leu Arg Ser
                100                     105                     110

Thr Asn Phe Ser Cys Leu Phe Val Asp Pro Gly Gln Val Ala Gln Tyr
                115                     120                     125

His Ile Ile Leu Ala Gln Leu Trp Asp Gly Leu Lys Thr Ala Pro Ser
        130                     135                     140

Pro Ser Gln Glu Thr Leu Ser Ser His Ser Pro Val Ser Arg Ser Ala

                    145                  150                  155                  160

Gly Pro Gly Val Ala

                                  165


(3) INFORMATION FOR SEQ ID NO: 3

        (i) SEQUENCE CHARACTERISTICS:

                (A) LENGTH: 22 amino acids

                (B) TYPE: amino acid

                (D) TOPOLOGY: linear

        (ii) MOLECULE TYPE: N-terminal fragment

        (Xi) SEQUENCE DESCRIPTION: SEQ ID NO: 3


Thr Pro Val Ser Gln Xaa Xaa Xaa Ala Ala Xaa Ala Xaa Val Arg Xaa

    1                5                        10                        15

Xaa Lys Asp Pro Cys Pro

                20


(4) INFORMATION FOR SEQ ID NO: 4

        (i) SEQUENCE CHARACTERISTICS:

                (A) LENGTH: 9 amino acids

                (B) TYPE: amino acid

                (D) TOPOLOGY: linear

        (ii) MOLECULE TYPE: internal fragment

        (Xi) SEQUENCE DESCRIPTION: SEQ ID NO: 4


Gly Ser Thr Gly Thr Gln Leu Cys Lys

    1                5

(5) INFORMATION FOR SEQ ID NO: 5

       (i) SEQUENCE CHARACTERISTICS:

           (A) LENGTH: 11 amino acids

           (B) TYPE: amino acid

           (D) TOPOLOGY: linear

       (ii) MOLECULE TYPE: internal fragment

       (Xi) SEQUENCE DESCRIPTION: SEQ ID NO: 5

Glu Arg Gly Ser Thr Gly Thr Gln Leu Cys Lys

(6) INFORMATION FOR SEQ ID NO: 6

       (i) SEQUENCE CHARACTERISTICS:

           (A) LENGTH: 8 amino acids

           (B) TYPE: amino acid

           (D) TOPOLOGY: linear

       (ii) MOLECULE TYPE: internal fragment

       (Xi) SEQUENCE DESCRIPTION: SEQ ID NO: 6

Leu Trp Glu Gly Ser Thr Ser Arg

(7) INFORMATION FOR SEQ ID NO: 7

       (i) SEQUENCE CHARACTERISTICS:

           (A) LENGTH: 15 amino acids

(B) TYPE: amino acid

(D) TOPOLOGY: linear

(ii) MOLECULE TYPE: N-terminal fragment

(Xi) SEQUENCE DESCRIPTION: SEQ ID NO: 7


Thr Pro Val Ser Gln Xaa Xaa Xaa Ala Ala Xaa Ala Xaa Val Arg
1               5                    10                    15


(8) INFORMATION FOR SEQ ID NO: 8

(i) SEQUENCE CHARACTERISTICS:

(A) LENGTH: 23 amino acids

(B) TYPE: amino acid

(D) TOPOLOGY: linear

(ii) MOLECULE TYPE: internal fragment

(Xi) SEQUENCE DESCRIPTION: SEQ ID NO: 8


His Val Val Leu Ala Gln Leu Trp Ala Gly Leu Arg Ala Xaa Leu Pro
1               5                    10                    15
Xaa Xaa Gln Glu Ala Leu Pro
             20


(9) INFORMATION FOR SEQ ID NO: 9

(i) SEQUENCE CHARACTERISTICS:

(A) LENGTH: 10 amino acids

(B) TYPE: amino acid

(D) TOPOLOGY: linear

(ii) MOLECULE TYPE: internal fragment

(Xi) SEQUENCE DESCRIPTION: SEQ ID NO: 9

Ala Leu Val Leu Glu Gln Leu Xaa Xaa Ala
1               5                  10

(10) INFORMATION FOR SEQ ID NO: 10

    (i) SEQUENCE CHARACTERISTICS:

        (A) LENGTH: 29 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 10

Ala Leu Val Leu Glu Gln Leu Thr Pro Ala Leu His Xaa Xaa Xaa Phe
1               5                  10                    15
Xaa Xaa Val Leu Val Asp Pro Glu Gln Val Val Gln Arg
        20                    25

(11) INFORMATION FOR SEQ ID NO: 11

    (i) SEQUENCE CHARACTERISTICS:

        (A) LENGTH: 12 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 11

Gln Cys Pro Ala Xaa Glu Val Thr Trp Xaa Glu Val

1                          5                          10

(12) INFORMATION FOR SEQ ID NO: 12

    (i) SEQUENCE CHARACTERISTICS:

      (A) LENGTH: 7 amino acids

      (B) TYPE: amino acid

      (D) TOPOLOGY: linear

    (ii) MOLECULE TYPE: internal fragment

    (Xi) SEQUENCE DESCRIPTION: SEQ ID NO: 12

Trp Glu Gly Ser Thr Ser Arg

1                          5

(13) INFORMATION FOR SEQ ID NO: 13

    (i) SEQUENCE CHARACTERISTICS:

      (A) LENGTH: 6 amino acids

      (B) TYPE: amino acid

      (D) TOPOLOGY: linear

    (ii) MOLECULE TYPE: internal fragment

    (Xi) SEQUENCE DESCRIPTION: SEQ ID NO: 13

Leu Val Asp Pro Glu Gln

1                          5

(14) INFORMATION FOR SEQ ID NO: 14

    (i) SEQUENCE CHARACTERISTICS:

(A) LENGTH: 7 amino acids

(B) TYPE: amino acid

(D) TOPOLOGY: linear

(ii) MOLECULE TYPE: internal fragment

(Xi) SEQUENCE DESCRIPTION: SEQ ID NO: 14


Ile Glu His Leu Pro Gly Arg
1               5


(15) INFORMATION FOR SEQ ID NO: 15

(i) SEQUENCE CHARACTERISTICS:

(A) LENGTH: 4 amino acids

(B) TYPE: amino acid

(D) TOPOLOGY: linear

(ii) MOLECULE TYPE: internal fragment

(Xi) SEQUENCE DESCRIPTION: SEQ ID NO: 15


His Val Val Leu
1


(16) INFORMATION FOR SEQ ID NO: 16

(i) SEQUENCE CHARACTERISTICS:

(A) LENGTH: 7 amino acids

(B) TYPE: amino acid

(D) TOPOLOGY: linear

(ii) MOLECULE TYPE: internal fragment

(Xi) SEQUENCE DESCRIPTION: SEQ ID NO: 16

Glu Gln Leu Thr Pro Ala Leu
1                    5

(17) INFORMATION FOR SEQ ID NO: 17

    (i) SEQUENCE CHARACTERISTICS:

        (A) LENGTH: 8 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 17

Ile Glu His Leu Pro Gly Arg Leu
1                    5

(18) INFORMATION FOR SEQ ID NO: 18

    (i) SEQUENCE CHARACTERISTICS:

        (A) LENGTH: 6 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 18

Tyr Xaa Leu Gly Xaa Gly
1                    5

22

(19) INFORMATION FOR SEQ ID NO: 19

    (i) SEQUENCE CHARACTERISTICS:

        (A) LENGTH: 4 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 19

Phe Pro Asn Phe
1

(20) INFORMATION FOR SEQ ID NO: 20

    (i) SEQUENCE CHARACTERISTICS:

        (A) LENGTH: 8 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 20

Tyr Xaa Leu Gly Xaa Gly Xaa Phe
1        5

(21) INFORMATION FOR SEQ ID NO: 21

    (i) SEQUENCE CHARACTERISTICS:

        (A) LENGTH: 7 amino acids

        (B) TYPE: amino acid

        (D) TOPOLOGY: linear

(ii) MOLECULE TYPE: internal fragment

(Xi) SEQUENCE DESCRIPTION: SEQ ID NO: 21

Glu Val Thr Xaa Xaa Glu Val

1                    5

(22) INFORMATION FOR SEQ ID NO: 22

    (i) SEQUENCE CHARACTERISTICS:

        (A) LENGTH: 8 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 22

Tyr Xaa Leu Gly Xaa Gly Xaa Phe

1                    5

(23) INFORMATION FOR SEQ ID NO: 23

    (i) SEQUENCE CHARACTERISTICS:

        (A) LENGTH: 11 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 23

Xaa Xaa Val Ala Xaa Xaa Arg Phe Pro Asn Phe

1                    5                    10

(24) INFORMATION FOR SEQ ID NO: 24

    (i) SEQUENCE CHARACTERISTICS:

        (A) LENGTH: 8 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 24

Leu Lys Glu Gly His Thr Ser Arg
1            5

(25) INFORMATION FOR SEQ ID NO: 25

    (i) SEQUENCE CHARACTERISTICS:

        (A) LENGTH: 11 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 25

Glu His Arg Xaa Thr Ser Thr Trp Leu His Arg
1           5           10

(26) INFORMATION FOR SEQ ID NO: 26

    (i) SEQUENCE CHARACTERISTICS:

        (A) LENGTH: 10 amino acids

(B) TYPE: amino acid

(D) TOPOLOGY: linear

(ii) MOLECULE TYPE: internal fragment

(Xi) SEQUENCE DESCRIPTION: SEQ ID NO: 26


Glu His Arg Xaa Thr Ser Thr Xaa Leu His
1               5               10



(27) INFORMATION FOR SEQ ID NO: 27

    (i) SEQUENCE CHARACTERISTICS:

        (A) LENGTH: 13 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 27


Xaa Xaa Xaa Xaa Xaa Xaa Xaa Xaa Ala Val Pro Thr Lys
1               5                   10



(28) INFORMATION FOR SEQ ID NO: 28

    (i) SEQUENCE CHARACTERISTICS:

        (A) LENGTH: 12 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 28

Ala Leu Val Leu Glu Glu Leu Ser Pro Thr Leu Arg

1                       5                       10


(29) INFORMATION FOR SEQ ID NO: 29

    (i) SEQUENCE CHARACTERISTICS:

        (A) LENGTH: 7 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 29


Ile Glu His Leu Pro Gly Arg

1                       5


(30) INFORMATION FOR SEQ ID NO: 30

    (i) SEQUENCE CHARACTERISTICS:

        (A) LENGTH: 6 amino acids

        (B) TYPE: amino acid

        (D) TOPOLOGY: linear

    (ii) MOLECULE TYPE: internal fragment

    (Xi) SEQUENCE DESCRIPTION: SEQ ID NO: 30


Xaa Asp Gly Leu Lys Thr

1                       5


(31) INFORMATION FOR SEQ ID NO: 31

(i) SEQUENCE CHARACTERISTICS:

    (A) LENGTH: 4 amino acids

    (B) TYPE: amino acid

    (D) TOPOLOGY: linear

(ii) MOLECULE TYPE: internal fragment

(Xi) SEQUENCE DESCRIPTION: SEQ ID NO: 31

His Ile Ile Leu
1

(32) INFORMATION FOR SEQ ID NO: 32

    (i) SEQUENCE CHARACTERISTICS:

        (A) LENGTH: 492 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: human

        (B) TISSUE TYPE: liver

    (ix) FEATURE:

        (A) NAME/KEY: mat peptide

        (B) LOCATION: 1..492

        (C) IDENTIFICATION METHOD: E

    (Xi) SEQUENCE DESCRIPTION: SEQ ID NO: 32

ACA CCT GTC TCG CAG ACC ACC ACA GCT GCC ACT GCC TCA GTT AGA AGC    48

Thr Pro Val Ser Gln Thr Thr Thr Ala Ala Thr Ala Ser Val Arg Ser

```
       1              5                    10                   15
ACA AAG GAC CCC TGC CCC TCC CAG CCC CCA GTG TTC CCA GCA GCT AAG        96
Thr Lys Asp Pro Cys Pro Ser Gln Pro Pro Val Phe Pro Ala Ala Lys

              20                   25                   30
CAG TGT CCA GCA TTG GAA GTG ACC TGG CCA GAG GTG GAA GTG CCA CTG       144
Gln Cys Pro Ala Leu Glu Val Thr Trp Pro Glu Val Glu Val Pro Leu

              35                   40                   45
AAT GGA ACG CTG AGC TTA TCC TGT GTG GCC TGC AGC CGC TTC CCC AAC       192
Asn Gly Thr Leu Ser Leu Ser Cys Val Ala Cys Ser Arg Phe Pro Asn

              50                   55                   60
TTC AGC ATC CTC TAC TGG CTG GGC AAT GGT TCC TTC ATT GAG CAC CTC       240
Phe Ser Ile Leu Tyr Trp Leu Gly Asn Gly Ser Phe Ile Glu His Leu
65                   70                   75                   80
CCA GGC CGA CTG TGG GAG GGG AGC ACC AGC CGG GAA CGT GGG AGC ACA       288
Pro Gly Arg Leu Trp Glu Gly Ser Thr Ser Arg Glu Arg Gly Ser Thr
                     85                   90                   95
GGT ACG CAG CTG TGC AAG GCC TTG GTG CTG GAG CAG CTG ACC CCT GCC       336
Gly Thr Gln Leu Cys Lys Ala Leu Val Leu Glu Gln Leu Thr Pro Ala

              100                  105                  110
CTG CAC AGC ACC AAC TTC TCC TGT GTG CTC GTG GAC CCT GAA CAG GTT       384
Leu His Ser Thr Asn Phe Ser Cys Val Leu Val Asp Pro Glu Gln Val

           115                  120                  125
GTC CAG CGT CAC GTC GTC CTG GCC CAG CTC TGG GCT GGG CTG AGG GCA       432
Val Gln Arg His Val Val Leu Ala Gln Leu Trp Ala Gly Leu Arg Ala

        130                  135                  140
ACC TTG CCC CCC ACC CAA GAA GCC CTG CCC TCC AGC CAC AGC AGT CCA       480
Thr Leu Pro Pro Thr Gln Glu Ala Leu Pro Ser Ser His Ser Ser Pro
145                  150                  155                  160
CAG CAG CAG GGT                                                        492
```

Gln Gln Gln Gly


(33) INFORMATION FOR SEQ ID NO: 33

        (i) SEQUENCE CHARACTERISTICS:

                (A) LENGTH: 495 base pairs

                (B) TYPE: nucleic acid

                (C) STRANDEDNESS: double

                (D) TOPOLOGY: linear

        (ii) MOLECULE TYPE: cDNA to mRNA

        (vi) ORIGINAL SOURCE:

                (A) ORGANISM: mouse

                (B) TISSUE TYPE: liver

        (ix) FEATURE:

                (A) NAME/KEY: mat peptide

                (B) LOCATION: 1..495

                (C) IDENTIFICATION METHOD: E

        (Xi) SEQUENCE DESCRIPTION: SEQ ID NO: 33


```
ACA TCT GCA CCT CAG ACA ACT GCC ACT GTC TTA ACT GGA AGC TCA AAA      48
Thr Ser Ala Pro Gln Thr Thr Ala Thr Val Leu Thr Gly Ser Ser Lys
 1               5                   10                  15
GAC CCA TGC TCT TCC TGG TCT CCA GCA GTC CCA ACT AAG CAG TAC CCA      96
Asp Pro Cys Ser Ser Trp Ser Pro Ala Val Pro Thr Lys Gln Tyr Pro
             20                  25                  30
GCA CTG GAT GTG ATT TGG CCA GAA AAA GAA GTG CCA CTG AAT GGA ACT     144
Ala Leu Asp Val Ile Trp Pro Glu Lys Glu Val Pro Leu Asn Gly Thr
         35                  40                  45
CTG ACC TTG TCC TGT ACT GCC TGC AGC CGC TTC CCC TAC TTC AGC ATC     192
```

Leu Thr Leu Ser Cys Thr Ala Cys Ser Arg Phe Pro Tyr Phe Ser Ile
50                    55                    60

CTC TAC TGG CTG GGC AAT GGT TCC TTC ATT GAG CAC CTT CCA GGC CGG    240
Leu Tyr Trp Leu Gly Asn Gly Ser Phe Ile Glu His Leu Pro Gly Arg
65              70                    75                    80

CTG AAG GAG GGC CAC ACA AGT CGC GAG CAC AGG AAC ACA AGC ACC TGG    288
Leu Lys Glu Gly His Thr Ser Arg Glu His Arg Asn Thr Ser Thr Trp
                85                    90                    95

CTG CAC AGG GCC TTG GTG CTG GAA GAA CTG AGC CCC ACC CTA CGA AGT    336
Leu His Arg Ala Leu Val Leu Glu Glu Leu Ser Pro Thr Leu Arg Ser
                100                   105                   110

ACC AAC TTC TCC TGT TTG TTT GTG GAT CCT GGA CAA GTG GCC CAG TAT    384
Thr Asn Phe Ser Cys Leu Phe Val Asp Pro Gly Gln Val Ala Gln Tyr
                115                   120                   125

CAC ATC ATT CTG GCC CAG CTC TGG GAT GGG TTG AAG ACA GCT CCG TCC    432
His Ile Ile Leu Ala Gln Leu Trp Asp Gly Leu Lys Thr Ala Pro Ser
                130                   135                   140

CCT TCT CAA GAA ACC CTC TCT AGC CAC AGC CCA GTA TCC AGA TCA GCA    480
Pro Ser Gln Glu Thr Leu Ser Ser His Ser Pro Val Ser Arg Ser Ala
145                   150                   155                   160

GGC CCA GGG GTT GCA                                                 495
Gly Pro Gly Val Ala
                165

(34) INFORMATION FOR SEQ ID NO: 34

    (i) SEQUENCE CHARACTERISTICS:

        (A) LENGTH: 411 base pairs

        (B) TYPE: nucleic acid

(C) STRANDEDNESS: double

(D) TOPOLOGY: linear

(ii) MOLECULE TYPE: cDNA to mRNA

(vi) ORIGINAL SOURCE:

    (A) ORGANISM: human

    (B) TISSUE TYPE: liver

(Xi) SEQUENCE DESCRIPTION: SEQ ID NO: 34


```
ACA CCT GTC TCG CAG ACC ACC ACA GCT GCC ACT GCC TCA GTT AGA AGC     48
Thr Pro Val Ser Gln Thr Thr Thr Ala Ala Thr Ala Ser Val Arg Ser
 1               5                   10                  15
ACA AAG GAC CCC TGC CCC TCC CAG CCC CCA GTG TTC CCA GCA GCT AAG     96
Thr Lys Asp Pro Cys Pro Ser Gln Pro Pro Val Phe Pro Ala Ala Lys
                20                  25                  30
CAG TGT CCA GCA TTG GAA GTG ACC TGG CCA GAG GTG GAA GTG CCA CTG    144
Gln Cys Pro Ala Leu Glu Val Thr Trp Pro Glu Val Glu Val Pro Leu
                35                  40                  45
AAT GGA ACG CTG AGC TTA TCC TGT GTG GCC TGC AGC CGC TTC CCC AAC    192
Asn Gly Thr Leu Ser Leu Ser Cys Val Ala Cys Ser Arg Phe Pro Asn
                50                  55                  60
TTC AGC ATC CTC TAC TGG CTG GGC AAT GGT TCC TTC ATT GAG CAC CTC    240
Phe Ser Ile Leu Tyr Trp Leu Gly Asn Gly Ser Phe Ile Glu His Leu
                65                  70                  75              80
CCA GGC CGA CTG TGG GAG GGG AGC ACC AGC CGG GAA CGT GGG AGC ACA    288
Pro Gly Arg Leu Trp Glu Gly Ser Thr Ser Arg Glu Arg Gly Ser Thr
                    85                  90                  95
GGT ACG CAG CTG TGC AAG GCC TTG GTG CTG GAG CAG CTG ACC CCT GCC    336
Gly Thr Gln Leu Cys Lys Ala Leu Val Leu Glu Gln Leu Thr Pro Ala
                100                 105                 110
```

CTG CAC AGC ACC AAC TTC TCC TGT GTG CTC GTG GAC CCT GAA CAG GTT  384

Leu His Ser Thr Asn Phe Ser Cys Val Leu Val Asp Pro Glu Gln Val

     115            120            125

GTC CAG CGT CAC GTC GTC CTG GCC CAG  411

Val Gln Arg His Val Val Leu Ala Gln

 130         135

(35) INFORMATION FOR SEQ ID NO: 35

    (i) SEQUENCE CHARACTERISTICS:

        (A) LENGTH: 216 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: human

        (B) TISSUE TYPE: liver

    (Xi) SEQUENCE DESCRIPTION: SEQ ID NO: 35

TGTGTGACTG GAGAAGAGGA CGTTGTCACA GATAAAGAGC CAGGCTCACC AGCTCCTGAC  60

GCATGCATC ATG ACC ATG AGA CAC AAC TGG ACA CCA GAC CTC AGC CCT TTG  111

        Met Thr Met Arg His Asn Trp Thr Pro Asp Leu Ser Pro Leu

        1          5             10

TGG GTC CTG CTC CTG TGT GCC CAC GTC GTC ACT CTC CTG GTC AGA GCC  159

Trp Val Leu Leu Leu Cys Ala His Val Val Thr Leu Leu Val Arg Ala

 15         20         25         30

ACA CCT GTC TCG CAG ACC ACC ACA GCT GCC ACT GCC TCA GTT AGA AGC  207

Thr Pro Val Ser Gln Thr Thr Thr Ala Ala Thr Ala Ser Val Arg Ser

                    35                    40                    45

ACA AAG GAC                                                              216

Thr Lys Asp


(36) INFORMATION FOR SEQ ID NO: 36

        (i) SEQUENCE CHARACTERISTICS:

            (A) LENGTH: 234 base pairs

            (B) TYPE: nucleic acid

            (C) STRANDEDNESS: double

            (D) TOPOLOGY: linear

        (ii) MOLECULE TYPE: cDNA to mRNA

        (vi) ORIGINAL SOURCE:

            (A) ORGANISM: human

            (B) TISSUE TYPE: liver

        (Xi) SEQUENCE DESCRIPTION: SEQ ID NO: 36


TTC TCC TGT GTG CTC GTG GAC CCT GAA CAG GTT GTC CAG CGT CAC GTC        48

Phe Ser Cys Val Leu Val Asp Pro Glu Gln Val Val Gln Arg His Val

  1               5                   10                  15

GTC CTG GCC CAG CTC TGG GCT GGG CTG AGG GCA ACC TTG CCC CCC ACC        96

Val Leu Ala Gln Leu Trp Ala Gly Leu Arg Ala Thr Leu Pro Pro Thr

              20                  25                  30

CAA GAA GCC CTG CCC TCC AGC CAC AGC AGT CCA CAG CAG CAG GGT           141

Gln Glu Ala Leu Pro Ser Ser His Ser Ser Pro Gln Gln Gln Gly

          35                  40                  45

TAAGACTCAG CACAGGGCCA GCAGCAGCAC AACCTTGACC AGAGCTTGGG TCCTACCTGT     201

CTACCTGGAG TGAACAGTCC CTGACTGCCT GTA                                  234

(37) INFORMATION FOR SEQ ID NO: 37

    (i) SEQUENCE CHARACTERISTICS:

        (A) LENGTH: 744 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: human

        (B) TISSUE TYPE: liver

    (ix) FEATURE:

        (A) NAME/KEY: mat peptide

        (B) LOCATION: 160..651

        (C) IDENTIFICATION METHOD: E

    (Xi) SEQUENCE DESCRIPTION: SEQ ID NO: 37


TGTGTGACTG GAGAAGAGGA CGTTGTCACA GATAAAGAGC CAGGCTCACC AGCTCCTGAC   60

GCATGCATC ATG ACC ATG AGA CAC AAC TGG ACA CCA GAC CTC AGC CCT TTG   111
        Met Thr Met Arg His Asn Trp Thr Pro Asp Leu Ser Pro Leu
        -30            -25            -20

TGG GTC CTG CTC CTG TGT GCC CAC GTC GTC ACT CTC CTG GTC AGA GCC   159
Trp Val Leu Leu Leu Cys Ala His Val Val Thr Leu Leu Val Arg Ala
    -15            -10            -5

ACA CCT GTC TCG CAG ACC ACC ACA GCT GCC ACT GCC TCA GTT AGA AGC   207
Thr Pro Val Ser Gln Thr Thr Thr Ala Ala Thr Ala Ser Val Arg Ser
    1            5            10           15

ACA AAG GAC CCC TGC CCC TCC CAG CCC CCA GTG TTC CCA GCA GCT AAG   255
Thr Lys Asp Pro Cys Pro Ser Gln Pro Pro Val Phe Pro Ala Ala Lys

```
              20                    25                    30
CAG TGT CCA GCA TTG GAA GTG ACC TGG CCA GAG GTG GAA GTG CCA CTG        303
Gln Cys Pro Ala Leu Glu Val Thr Trp Pro Glu Val Glu Val Pro Leu
              35                    40                    45
AAT GGA ACG CTG AGC TTA TCC TGT GTG GCC TGC AGC CGC TTC CCC AAC        351
Asn Gly Thr Leu Ser Leu Ser Cys Val Ala Cys Ser Arg Phe Pro Asn
              50                    55                    60
TTC AGC ATC CTC TAC TGG CTG GGC AAT GGT TCC TTC ATT GAG CAC CTC        399
Phe Ser Ile Leu Tyr Trp Leu Gly Asn Gly Ser Phe Ile Glu His Leu
  65                    70                    75                    80
CCA GGC CGA CTG TGG GAG GGG AGC ACC AGC CGG GAA CGT GGG AGC ACA        447
Pro Gly Arg Leu Trp Glu Gly Ser Thr Ser Arg Glu Arg Gly Ser Thr
                    85                    90                    95
GGT ACG CAG CTG TGC AAG GCC TTG GTG CTG GAG CAG CTG ACC CCT GCC        495
Gly Thr Gln Leu Cys Lys Ala Leu Val Leu Glu Gln Leu Thr Pro Ala
              100                   105                   110
CTG CAC AGC ACC AAC TTC TCC TGT GTG CTC GTG GAC CCT GAA CAG GTT        543
Leu His Ser Thr Asn Phe Ser Cys Val Leu Val Asp Pro Glu Gln Val
              115                   120                   125
GTC CAG CGT CAC GTC GTC CTG GCC CAG CTC TGG GCT GGG CTG AGG GCA        591
Val Gln Arg His Val Val Leu Ala Gln Leu Trp Ala Gly Leu Arg Ala
              130                   135                   140
ACC TTG CCC CCC ACC CAA GAA GCC CTG CCC TCC AGC CAC AGC AGT CCA        639
Thr Leu Pro Pro Thr Gln Glu Ala Leu Pro Ser Ser His Ser Ser Pro
145                   150                   155                   160
CAG CAG CAG GGT TAAGACTCAG CACAGGGCCA GCAGCAGCAC AACCTTGACC            691
Gln Gln Gln Gly

AGAGCTTGGG TCCTACCTGT CTACCTGGAG TGAACAGTCC CTGACTGCCT GTA            744
```

(38) INFORMATION FOR SEQ ID NO: 38

    (i) SEQUENCE CHARACTERISTICS:

        (A) LENGTH: 351 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: mouse

        (B) TISSUE TYPE: liver

    (Xi) SEQUENCE DESCRIPTION: SEQ ID NO: 38


```
GCA GTC CCA ACT AAG CAG TAC CCA GCA CTG GAT GTG ATT TGG CCA GAA     48
Ala Val Pro Thr Lys Gln Tyr Pro Ala Leu Asp Val Ile Trp Pro Glu
 1               5                   10                  15
AAA GAA GTG CCA CTG AAT GGA ACT CTG ACC TTG TCC TGT ACT GCC TGC     96
Lys Glu Val Pro Leu Asn Gly Thr Leu Thr Leu Ser Cys Thr Ala Cys
                20                  25                  30
AGC CGC TTC CCC TAC TTC AGC ATC CTC TAC TGG CTG GGC AAT GGT TCC    144
Ser Arg Phe Pro Tyr Phe Ser Ile Leu Tyr Trp Leu Gly Asn Gly Ser
                35                  40                  45
TTC ATT GAG CAC CTT CCA GGC CGG CTG AAG GAG GGC CAC ACA AGT CGC    192
Phe Ile Glu His Leu Pro Gly Arg Leu Lys Glu Gly His Thr Ser Arg
                50                  55                  60
GAG CAC AGG AAC ACA AGC ACC TGG CTG CAC AGG GCC TTG GTG CTG GAA    240
Glu His Arg Asn Thr Ser Thr Trp Leu His Arg Ala Leu Val Leu Glu
65                  70                  75                  80
GAA CTG AGC CCC ACC CTA CGA AGT ACC AAC TTC TCC TGT TTG TTT GTG    288
```

Glu Leu Ser Pro Thr Leu Arg Ser Thr Asn Phe Ser Cys Leu Phe Val

          85              90              95

GAT CCT GGA CAA GTG GCC CAG TAT CAC ATC ATT CTG GCC CAG CTC TGG    336

Asp Pro Gly Gln Val Ala Gln Tyr His Ile Ile Leu Ala Gln Leu Trp

         100           105          110

GAT GGG TTG AAG ACA                              351

Asp Gly Leu Lys Thr

       115

(39) INFORMATION FOR SEQ ID NO: 39

    (i) SEQUENCE CHARACTERISTICS:

        (A) LENGTH: 336 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: mouse

        (B) TISSUE TYPE: liver

    (xi) SEQUENCE DESCRIPTION: SEQ ID NO: 39

CTGAGCCTTA GAGCTCCAAG AAGCTATTCG GGGCTTAGGA GCCAGAAGCT GACTGCTGCC    60

TGCCCTTCCC AGAAGGAGGC TGGCAAGCTG GCAAACGGAC TGTTGCTTCC CAGAGGAAGT    120

CACAGACACC AGACTTGCTT GCAAGTCATC ATG ACC ATG AGA CAC TGC TGG ACA    174

                                        Met Thr Met Arg His Cys Trp Thr

                                        1           5

GCA GGC CCC AGT TCT TGG TGG GTC CTG CTT TTG TAT GTC CAT GTC ATT    222

Ala Gly Pro Ser Ser Trp Trp Val Leu Leu Leu Tyr Val His Val Ile

```
                10              15              20
TTG GCC AGA GCC ACA TCT GCA CCT CAG ACA ACT GCC ACT GTC TTA ACT    270
Leu Ala Arg Ala Thr Ser Ala Pro Gln Thr Thr Ala Thr Val Leu Thr
    25              30              35              40
GGA AGC TCA AAA GAC CCA TGC TCT TCC TGG TCT CCA GCA GTC CCA ACT    318
Gly Ser Ser Lys Asp Pro Cys Ser Ser Trp Ser Pro Ala Val Pro Thr
                    45              50              55
AAG CAG TAC CCA GCA CTG                                            336
Lys Gln Tyr Pro Ala Leu
                    60
```

(40) INFORMATION FOR SEQ ID NO: 40

    (i) SEQUENCE CHARACTERISTICS:

        (A) LENGTH: 253 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: mouse

        (B) TISSUE TYPE: liver

    (xi) SEQUENCE DESCRIPTION: SEQ ID NO: 40

```
GAT CCT GGA CAA GTG GCC CAG TAT CAC ATC ATT CTG GCC CAG CTC TGG    48
Asp Pro Gly Gln Val Ala Gln Tyr His Ile Ile Leu Ala Gln Leu Trp
1               5               10              15
GAT GGG TTG AAG ACA GCT CCG TCC CCT TCT CAA GAA ACC CTC TCT AGC    96
Asp Gly Leu Lys Thr Ala Pro Ser Pro Ser Gln Glu Thr Leu Ser Ser
```

```
              20                25                30
CAC AGC CCA GTA TCC AGA TCA GCA GGC CCA GGG GTT GCA TAAAGCCAAC    145
His Ser Pro Val Ser Arg Ser Ala Gly Pro Gly Val Ala
              35                40                45
CACACCATGA CCTTGACCAG AGCCTGGCTC TCATCTACCT GGAGGGTGGA GTCTACACCA  205
TAGGCTGTGA TTGCCTTTCT GCTGCTGAAC CTCAAACTCA AGCTTCAC              253
```

(41) INFORMATION FOR SEQ ID NO: 41

    (i) SEQUENCE CHARACTERISTICS:

        (A) LENGTH: 847 base pairs

        (B) TYPE: nucleic acid

        (C) STRANDEDNESS: double

        (D) TOPOLOGY: linear

    (ii) MOLECULE TYPE: cDNA to mRNA

    (vi) ORIGINAL SOURCE:

        (A) ORGANISM: mouse

        (B) TISSUE TYPE: liver

    (ix) FEATURE:

        (A) NAME/KEY: mat peptide

        (B) LOCATION: 235..729

        (C) IDENTIFICATION METHOD: E

    (xi) SEQUENCE DESCRIPTION: SEQ ID NO: 41

```
CTGAGCCTTA GAGCTCCAAG AAGCTATTCG GGGCTTAGGA GCCAGAAGCT GACTGCTGCC   60
TGCCCTTCCC AGAAGGAGGC TGGCAAGCTG GCAAACGGAC TGTTGCTTCC CAGAGGAAGT  120
CACAGACACC AGACTTGCTT GCAAGTCATC ATG ACC ATG AGA CAC TGC TGG ACA   174
                                  Met Thr Met Arg His Cys Trp Thr
```

GCA GGC CCC AGT TCT TGG TGG GTC CTG CTT TTG TAT GTC CAT GTC ATT    222

Ala Gly Pro Ser Ser Trp Trp Val Leu Leu Leu Tyr Val His Val Ile

-20          -15          -10          -5

TTG GCC AGA GCC ACA TCT GCA CCT CAG ACA ACT GCC ACT GTC TTA ACT    270

Leu Ala Arg Ala Thr Ser Ala Pro Gln Thr Thr Ala Thr Val Leu Thr

1          5          10

GGA AGC TCA AAA GAC CCA TGC TCT TCC TGG TCT CCA GCA GTC CCA ACT    318

Gly Ser Ser Lys Asp Pro Cys Ser Ser Trp Ser Pro Ala Val Pro Thr

15          20          25

AAG CAG TAC CCA GCA CTG GAT GTG ATT TGG CCA GAA AAA GAA GTG CCA    366

Lys Gln Tyr Pro Ala Leu Asp Val Ile Trp Pro Glu Lys Glu Val Pro

30          35          40

CTG AAT GGA ACT CTG ACC TTG TCC TGT ACT GCC TGC AGC CGC TTC CCC    414

Leu Asn Gly Thr Leu Thr Leu Ser Cys Thr Ala Cys Ser Arg Phe Pro

45          50          55          60

TAC TTC AGC ATC CTC TAC TGG CTG GGC AAT GGT TCC TTC ATT GAG CAC    462

Tyr Phe Ser Ile Leu Tyr Trp Leu Gly Asn Gly Ser Phe Ile Glu His

65          70          75

CTT CCA GGC CGG CTG AAG GAG GGC CAC ACA AGT CGC GAG CAC AGG AAC    510

Leu Pro Gly Arg Leu Lys Glu Gly His Thr Ser Arg Glu His Arg Asn

80          85          90

ACA AGC ACC TGG CTG CAC AGG GCC TTG GTG CTG GAA GAA CTG AGC CCC    558

Thr Ser Thr Trp Leu His Arg Ala Leu Val Leu Glu Glu Leu Ser Pro

95          100          105

ACC CTA CGA AGT ACC AAC TTC TCC TGT TTG TTT GTG GAT CCT GGA CAA    606

Thr Leu Arg Ser Thr Asn Phe Ser Cys Leu Phe Val Asp Pro Gly Gln

110          115          120

GTG GCC CAG TAT CAC ATC ATT CTG GCC CAG CTC TGG GAT GGG TTG AAG    654

Val Ala Gln Tyr His Ile Ile Leu Ala Gln Leu Trp Asp Gly Leu Lys

```
        125                 130                 135                 140
ACA GCT CCG TCC CCT TCT CAA GAA ACC CTC TCT AGC CAC AGC CCA GTA    702
Thr Ala Pro Ser Pro Ser Gln Glu Thr Leu Ser Ser His Ser Pro Val
                    145                 150                 155
TCC AGA TCA GCA GGC CCA GGG GTT GCA TAAAGCCAAC CACACCATGA          749
Ser Arg Ser Ala Gly Pro Gly Val Ala
                    160                 165
CCTTGACCAG AGCCTGGCTC TCATCTACCT GGAGGGTGGA GTCTACACCA TAGGCTGTGA  809
TTGCCTTTCT GCTGCTGAAC CTCAAACTCA AGCTTCAC                          847
```

**Description**

TECHNICAL FIELD

This invention relates to a novel cytokine-binding protein, particularly, an interleukin-18-binding protein.

BACKGROUND ART

Interleukin-18 (hereinafter abbreviated as "IL-18") is a type of cytokine that transduces signals in immune system. As documented in Japanese Patent Kokai Nos. 27,189/96 and 193,098/96 and Haruki Okamura et al., *"Nature, "* Vol. 378, No. 6552, pp.88-91 (1995), IL-18 was designated "interferon-γ inducing factor (IGIF)" immediately after its discovery; this designation was changed later into "IL-18 (interleukin-18)" in accordance with the proposal in Shimpei Ushio et al., *"The Journal of Immunology,"* Vol.156, pp.4274-4279 (1996). As described in *"The Cytokine Handbook,"* edited by Angus W. Thomson, published by Academic Press Ltd.(1998), pp.465-489, mature IL-18 consists of 157 amino acids and has the activities of inducing the production of interferon-γ (hereinafter abbreviated as "IFN-γ"), which is useful as a physiologically active protein, by immunocompetent cells, as well as of enhancing the cytotoxicity of killer cells and inducing the generation of killer cells. Because of these activities, IL-18 has been deemed useful in various pharmaceuticals, for example, an anti-viral agent, anti-microbial agent, anti-tumor agent, and anti-immunopathic agent. Energetic studies are now in progress to realize these potential uses.

As mentioned above, IL-18, like other cytokines, is inherently produced and secreted as a substance responsible for signal transduction in immune system. Therefore, excessive amounts of IL-18 may disturb the balance of immune system when over-produced or excessively administered in the body of mammals. Recent studies have demonstrated that patients with autoimmune diseases including rheumatoid arthritis are significantly higher in IL-18 level in their body fluids than healthy humans, as disclosed in Japanese Patent Kokai No.96730/98. This indicates the possibility that IL-18 directly or indirectly relates to the crisis of certain diseases. In this field, as well as for the clarification in physiological activities and practical utilization of IL-18, there is a great demand for earlier clarification and utilization of a substance which suppresses the physiological activities of IL-18.

In view of the foregoing, the first object of this invention is to provide a substance which is capable of suppressing the physiological activities of IL-18 and applicable to humans and other mammals.

The second object of this invention is to provide a DNA encoding the substance.

The third object of this invention is to provide uses of the substance as an IL-18-suppressor.

The fourth object of this invention is to provide uses of the substance as a pharmaceutical.

DISCLOSURE OF INVENTION

The present inventors energetically studied to attain the above objects. As a result of theses studies, the inventors found a substance in mammalian body fluids which suppresses the physiological activities of IL-18 through binding to IL-18. The inventors then isolated this substance and investigated for its characteristics and properties. This substance was proved in the nature of a protein, and exhibited the ability of binding to IL-18 and thus suppressing the physiological activities thereof even in the isolated form. Further, this IL-18-binding protein, thus identified, was found to have an efficacy in treatment and prevention of various diseases resulting from augmented immunoreactions such as autoimmune diseases, inflammatory diseases, and allergic diseases, when administered to humans and other mammals.

Specifically, this invention attains the first object by providing the IL-18-binding protein comprising a part or the whole of the amino acid sequence shown in SEQ ID NO:1 or 2.

This invention attains the second object by providing a DNA encoding this IL-18-binding protein.

This invention attains the third object by providing an IL-18-suppressor containing as an effective ingredient this IL-18-binding protein.

This invention attains the fourth object by providing an agent for susceptive diseases containing as an effective ingredient this IL-18-binding protein.

BRIEF DESCRIPTION OF DRAWINGS

FIG. 1. shows peptide maps of the IL-18-binding protein of human origin.

FIG. 2. shows peptide maps of the IL-18-binding protein of mouse origin.

FIG. 3. shows a restriction enzyme map of a recombinant DNA comprising a nucleotide sequence encoding the IL-18-binding protein of human origin.

FIG. 4. shows a restriction enzyme map of a recombinant DNA comprising a nucleotide sequence encoding the IL-18-binding protein of mouse origin.

In the figures, the meanings of the symbols are as follows:

EFH18BPH6 cDNA, cDNA comprising a nucleotide sequence encoding the IL-18-binding protein of human origin;
EFM18BPH-MK2 cDNA, cDNA comprising a nucleotide sequence encoding the IL-18-binding protein of mouse origin;
EF1αP, elongation factor 1 promotor;
Amp, ampicillin-resistant gene; and
ori, replication origin.

BEST MODE OF INVENTION

The following are to explain the best mode of this invention; the protein of this invention is characterized by the property of suppressing the physiological activities of IL-18 through binding to IL-18 and by its specific amino acid sequences. The IL-18-binding protein of this invention, when acting on IL-18, suppresses the representative physiological activity of IL-18, inducing IFN-γ production by immunocompetent cells. Further, the IL-18-binding protein of this invention, when binding to IL-18, may suppress the enhancement of cytotoxicity of killer cells and the induction of killer cell generation by the action of IL-18. The IL-18-binding protein of this invention comprises a part or the whole of the amino acid sequence shown in SEQ ID NO:1 or 2 in the sequence listing; for example, the IL-18-binding protein of human origin comprises as a partial amino acid sequence(s) a part or the whole of the amino acid sequence shown in at least one of SEQ ID NOs:3 to 23, and the IL-18 binding protein of mouse origin comprises as a partial amino acid sequence(s) a part or the whole of the amino acid sequences shown in at least one of SEQ ID NOs:24 to 31. In body fluids such as urine and blood, the IL-18-binding protein of this invention usually exists as a soluble protein, which exhibits, on SDS-polyacrylamide gel electrophoresis, a protein band bearing IL-18-binding ability at a molecular weight of about 40,000 to about 60,000 daltons.

The IL-18-binding protein of this invention can be obtained from mammalian body fluids and cells by studying them for the above characteristics as criteria. The body fluids include bloods, lymphs, ascites, and urines, and the cells include epidermal cells, endothelial cells, interstitial cells, chondrocytes, monocytes, lymphocytes, neurocytes, and cell lines establishable from these cells. With regard to cost for preparation, it is advantageous to apply recombinant DNA techniques with a DNA encoding the IL-18-binding protein of this invention. DNAs encoding the IL-18-binding protein of this invention can be obtained by screening mammalian genes on the basis of the amino acid sequences shown in SEQ ID NOs:1 to 31. A DNA of human origin encoding the IL-18-binding protein of this invention usually comprises a part or the whole of the nucleotide sequence shown in SEQ ID NO:32, and a DNA of mouse origin usually comprises a part or the whole of the nucleotide sequence shown in SEQ ID NO:33. Mammalian or microbial host cells transformed with such DNAs can produce the IL-18-binding protein of this invention at relatively high yields, when the cells are cultured in a usual manner. The mammalian host cells include, for example, 3T3 cells (ATCC CCL-92), C127I cells (ATCC CRL-1616), CHO-K1 cells (ATCC CCL-61), CV-1 cells (ATCC CCL-70), COS-1 cells (ATCC CRL-1650), HeLa cells (ATCC CCL-2), MOP 8 cells (ATCC CRL-1709), mutant strains from these cells, and other epidermal cells, interstitial cells, and hemopoietic cells of human, monkey, mouse, or hamster origin. The microbial host cells include, for example, bacteria, fungi, and yeasts. Among these host cells, mammalian host cells and yeasts are more advantageous for the production of the IL-18-binding protein in the form of a glycoprotein.

To prepare the IL-18-binding protein of this invention from the sources as described above, the body fluids or the cellular or microbial cultures can be disrupted if necessary, for example, by sonication, and then subjected to conventional methods to purify physiologically active proteins. The conventional methods include salting-out, dialysis, filtration, concentrating, separatory sedimentation, ion-exchange chromatography, gel filtration chromatography, adsorption chromatography, isoelectric focusing chromatography, hydrophobic chromatography, reversed phase chromatography, affinity chromatography, gel electrophoresis, and isoelectric focusing electrophoresis, which can be applied alone or in combination.

Immune system inherently functions to protect a living body from foreign noxious substances, but under certain conditions, this function rather causes injurious affections to the living body. In the case of organ transplantation such as grafting skins, kidneys, livers, hearts, bone marrows to mammals, rejection reactions against alloantigens may activate T cells, induce lymphocyte proliferation, and then cause inflammation. While differently in symptoms, similar phenomena can be observed in the case of invasion of exogenous antigens such as allergens that a host recognizes as non-self. In autoimmune diseases, substances that should be recognized as self by a host induce allergic reactions.

Because the IL-18-binding protein of this invention functions as an agent to suppress the physiological activities of IL-18 through binding to IL-18, which is responsible for activation of immune system, the protein of this invention is expected to suppress immunoreactions as described above when administered to humans and other mammals. Therefore, the term "susceptive diseases" as referred to in this invention includes immunopathies resulting from aug-

**EP 1 110 969 A1**

mented immunoreactions in general, such as rejection reactions and allergic reactions, and the diseases that can be treated or prevented by the direct or indirect action of the IL-18-binding protein of this invention. The susceptive diseases include, for example, the above-mentioned rejection reactions associated with organ transplantation, active chronic hepatitis, atrophic gastritis, autoimmune hemolytic anemia, Basedow's disease, Behcet's syndrome, CRST syndrome, cold agglutination hemolytic anemia, ulcerative colitis, Goodpasture's syndrome, hyperthyroidism, chronic thyroiditis, idiopathic thrombocytopenic purpura, juvenile diabetes, leukopenia, multiple sclerosis, severe myasthenia, paroxysmal cold hemoglobinuria, pernicious anemia, polyarteritis nodosa, multiple myositis, primary biliary cirrhosis, rheumatic fever, rheumatoid arthritis, Hashimoto's disease, Sjögren's syndrome, Crohn's disease, sympathetic ophthalmia, progressive systemic sclerosis, Wegener's granulomatosis, HIV infection, asthma, atopic dermatitis, allergic rhinitis, pollinosis, apitoxin allergy, and other autoimmune, inflammatory, and allergic diseases in general. The IL-18-binding protein of this invention has another efficacy to treat or prevent septic shock resulting from excessively produced or administered IFN-$\gamma$. In a living body, IL-18 possibly augments Fas-ligand production, and inversely, Fas-ligand possibly induces IL-18 secretion from cells. The IL-18-binding protein is therefore efficacious in treatment and prevention of immunopathies relating to Fas and to Fas-ligand in general. In addition, the IL-18-binding protein of this invention is efficacious in treatment or prevention of hepatic disorders such as viral hepatitis, alcoholic hepatitis, toxic hepatitis, fulminant hepatitis, viral cirrhosis, alcoholic cirrhosis, toxic cirrhosis, biliary cirrhosis, fatty liver, hepatic tumors, and hepatic angiopathies, cholesystopathies or biliary disorders such as cholangitis, cholecystitis, primary sclerosing cholangitis, cholecystic tumors, and biliary tumors, pancreatopathies such as acute pancreatitis, chronic pancreatitis, deficiency in pancreatic functions, pancreatic tumors, and hydrocyst, as well as in alleviation or improvement of symptoms associated with these disorders, for example, inappetence, malaise, fatigue, bellyache, dorsalgia, icterus, fever, hepatic encephalosis, ascites, hemorrhagic determination, and other dyshepatia and hepatargia. In these cases, a medicament(s) capable of activating hepatic functions such as protoporphyrin, thioprine, malotilate, liver hydrolyzates, glycyrrhizin, dichloroacetate diisopropylamine, methylmethionine sulfonium chloride, glutathione, taurine, cyanidanol, interferons, vitamin B1, vitamin B2, vitamin B6, vitamin B12, thioctic acid, *hsiao-tzŭ-ku-t'ang*, *ta-tzŭ-ku-t'ang*, *tzŭ-ku-kuei-chih-t'ang*, aspartic acid, glycyrrhiza, methionine, thioprine, and glycyrrhizin can be used in combination. The IL-18-binding protein further additionally has an efficacy to alleviate or prevent disorders in circulatory system such as ischemia, ischemic cardiomyopathy, cerebral ischemia, basilar artery migraine, abnormal vascularnet at the brain base, cerebral apoplexy, aneurysm at the brain base, arteriosclerosis, disorders in vascular endothelium, diabetes, mesenteric angiemphraxis, and superior mesenteric artery syndrome and disorders in nerve system such as Parkinson's disease, spinomuscular amyotrophy, amyotrophic sclerosis at the funiculus lateralis, Alzheimer's disease, dementia, cerebrovascular dementia, AIDS dementia, and encephalomyelitis. As above, the agent for susceptive diseases of this invention, containing the IL-18-biding protein as an effective ingredient, has a variety of uses to treat or prevent the above-mentioned susceptive diseases, for example, as an anti-autoimmune agent, anti-inflammatory agent, anti-allergic agent, anti-tumor agent, immunosuppressant, hemopoietic agent, thrombopoietic agent, lenitive agent, antipyretic agent, and agent to improve hepatic functions. The agent for susceptive diseases of this invention is usually prepared in the form of a liquid, suspension, paste, or solid, and contains the IL-18-binding protein of this invention in a content of 0.00001-100%(w/w), preferably, 0.0001-20%(w/w), while the content may vary depending on the form of this agent as well as the types and symptoms of the susceptive diseases to be treated.

The agent for susceptive diseases of this invention includes those in the form consisting of the IL-18-binding protein of this invention alone and in the form of a composition comprising this protein and one or more of other physiologically acceptable, for example, adjuvants, extenders, diluents, excipients, stabilizers, antiseptics, immuno-adjuvants, colors, flavors, and if necessary, physiologically active substances. The stabilizers include following examples: proteins such as serum albumen and gelatins; saccharides such as glucose, sucrose, lactose, maltose, trehalose, sorbitol, maltitol, mannitol, and lactitol; and buffers mainly composed of citrates, phosphates, or carbonates. The physiologically active substances usable in combination include following examples: anti-inflammatory agents such as aspirin, flufenamic acid, mefenamic acid, diclofenac, indomethacin, tolmetin, ibuprofen, ketoprofen, phenylbutazone, oxyphenbutazone, anti-inflammatory enzyme preparations, gold preparations, and chloroquine preparations; immunosuppressants such as FK506, cyclophosphamide, azathioprine, methotrexate, cyclosporin A, and adrenal cortical hormones; and further, antagonists against receptors for IL-18 and other cytokines, for example, antibodies including humanized antibodies respectively against interleukin-1-receptor protein, interleukin-2-receptor protein, interleukin-5-receptor protein, interleukin-6-receptor protein, interleukin-8-receptor protein, interleukin-12-receptor protein, and IL-18-receptor protein; antagonists respectively against TNF-$\alpha$, TNF-$\beta$, interleukin-1-receptor, interleukin-5-receptor, interleukin-8-receptor, and IL-18-receptor; and antibodies including humanized antibodies respectively against interleukin-1, interleukin-2, interleukin-5, interleukin-8, interleukin-6, interleukin-8, interleukin-12, and interleukin-18.

The agent for susceptive diseases of this invention further includes pharmaceutics in the form for a single shot of medication. The pharmaceutics in such form contain the IL-18-binding protein, for example, in a content corresponding to multiples (up to fourfold) or divisor (not less than 1/40) of its single dosage, in a physically united formula suitable for medication. The formulae of such pharmaceutics include extracts, elixirs, capsules, granules, pills, oph-

4

thalmic ointments, suspensions, emulsions, plasters, suppositories, powders, spirits, tablets, syrups, infusions, decoctions, injections, replacement fluids, tinctures, ophthalmic solutions, troches, ointments, cataplasmas, aromatic waters, liniments, lemonades, fluidextracts, and lotions, and if necessary, nasal drops, nasal sprays, inhalations for lower airway, sustained release preparations for ophthalmic treatment, plastering tablets for tunica mucosa oris, and clysters. The agent for susceptive diseases of this invention can be administered orally and parenterally; both the administrations can effectively treat or prevent the susceptive diseases. The agent of this invention can be administered to patients usually in accordance with the symptom of each patient observed before and/or after treatment, for example, at a dosage for adult humans of about 1 μg/shot to 1 g/shot, usually, about 10 μg/shot to 100 mg/shot, with a frequency of 1 to 4 shot/day or 1 to 5 shot/week over 1 day to half a year through oral route or parenteral route such as intracutaneous, subcutaneous, intramuscular, and intravenous routes.

The DNAs encoding the IL-18-binding protein of this invention are useful also in so-called "gene therapies." In conventional gene therapies, the DNA of this invention can be inserted into a viral vector such as retroviral vector, adenoviral vector, and adeno-associated-viral vector, or incorporated in a liposome such as cationic polymer and membrane-fused liposome, and in such form, the DNA can be directly injected into patients with diseases susceptive to the IL-18-binding protein. Alternatively, into lymphocytes collected from such patients, the DNA of this invention can be introduced *in vitro,* and the lymphocytes can be autografted to the patients. Thus the DNAs of this invention exhibit a distinguished efficacy in gene therapies for immunopathies such as autoimmune diseases, allergic diseases, and other diseases including liver disorders and nerve system disorders, as well as in suppression of rejection reactions and excessive immunoreactions associated with organ transplantation. General procedures for the gene therapies as above are detailed, for example, in "*Jikken-Igaku-Bessatsu*, *Bio-manual Up Series, Idenshichiryo-no-Kisogijutsu* (Basic Techniques for Gene Therapy)," edited by Takashi Shimada, Izumi Saito, and Toshiya Ozawa, published by Yodosha (1996).

The following are to explain the preferred embodiments of this invention in line with Examples, while these Examples can be variously modified by the level of techniques in this field. In view of this, this invention should not be restricted to these Examples only. In following Examples, IL-18-binding ability was judged by percent inhibition as a criteria determinable by the binding assay as follows.

As effector cells, cells expressing IL-18 receptor abundantly on the surface thereof are prepared by introduction of a DNA encoding IL-18 receptor into CHO-K1 cells (ATCC CRL-9618), derived from Chinese hamster ovary. As an assay medium, RPMI-1640 medium (pH 7.2) containing 0.1%(w/v) sodium azide, 0.1%(v/v) bovine serum albumin, and 100 mM N-2-hydroxyethylpiperazine-N'-2-ethane sulfonic acid is prepared. In a system for test, 50 μl of a test sample appropriately diluted with the assay medium is admixed with 50 μl of $^{125}$I-labeled IL-18 appropriately diluted with the assay medium, and shaken at 4°C for 1 hour. This mixture is then admixed with 50 μl of a suspension of the effector cells in the assay medium having a cell density of $1 \times 10^7$ cells/ml, and shaken at 4°C for another 1 hour. Thereafter, the resultant suspension of the effector cells is overlaid on 200 μl of a mixture of dibutyl phthalate and dioctyl phthalate (1:1 by volume) poured in 1.5-ml centrifugal tube, and then centrifuged at 4 C for 5 minutes. The supernatant is removed by aspiration. The residual cells are cut out together with the tube, and measured for radio activity by gamma counter ("Type ARC-300," produced by Aloka Co., Ltd.). Further, a system (for non-specific binding) in which 5 μg of non-labeled IL-18 is added together with $^{125}$I-labeled IL-18 and another system (for total binding) with no test sample are treated similarly as in the test system. The measured radio activities, in the systems for test, total binding, and non-specific binding, are introduced into the following equation to calculate percent inhibition (%).

$$\text{Percent Inhibition (\%)} = \frac{\text{(Total Binding) - (Test)}}{\text{(Total Binding) - (Non-Specific Binding)}} \times 100$$

Example 1: IL-18-binding protein of human origin

Example 1-1: Preparation of IL-18-binding protein

Three liters of human urine was concentrated with a membrane, and dialyzed against 20 mM phosphate buffer (pH 7.0) at 4°C for 20 hours. The dialyzed liquid was collected, and then applied to a column with 230 ml of affinity chromatography gel ("Wheat Germ Lectin Sepharose 6MB," commercialized by Amersham Pharmacia Biotech Co., Ltd.), which had been equilibrated with 20 mM phosphate buffer (pH 7.0), to adsorb the IL-18-binding protein. The column was washed with 20 mM phosphate buffer (pH 7.0), and 20 mM phosphate buffer (pH 7.0) containing 0.5 M N-acetyl-D-glucosamine was then fed to the column while the liquid eluted from the column was fractionated by a prescribed volume.

The eluted fractions were examined for IL-18-binding ability by the above-described binding assay. Fractions in which IL-18-binding property was observed were pooled and dialyzed against 20 mM phosphate buffer (pH 7.0) at 4°C for 16 hours. The dialyzed liquid was collected, concentrated to a prescribed volume, and then applied to a column with 54 ml of ion-exchange chromatography gel ("TSK-gel DEAE-5PW," produced by TOSO Co., Ltd.), which had been equilibrated with 20 mM phosphate buffer (pH 7.0). To the column, 20 mM phosphate buffer (pH 7.0) containing sodium chloride was fed at a flow rate of 2 ml/min while the sodium chloride concentration was controlled to increase from 0 to 0.5 M over 100 minutes in a linear gradient manner. A fraction eluted at about 0.2 M sodium chloride was collected.

The above fraction was membrane-concentrated, and then applied to a column with 120 ml of gel-filtration chromatography gel ("HilLoad Superdex 200," Amersham Pharmacia Biotech Co., Ltd.), which had been equilibrated with 20 mM phosphate-beffered saline (hereinafter abbreviated as "PBS"). To the column PBS was fed, and a fraction corresponding to a molecular weight of about 70,000 daltons on this gel filtration chromatography was collected. This newly obtained fraction was applied to a column with 4 ml of reversed phase chromatography gel ("Vydac 214TP54," commercialized by Cypress International, Ltd.), which had been equilibrated with 0.1%(v/v) trifluoroacetic acid. To the column, 0.1%(v/v) trifluoroacetic acid containing acetonitrile was fed while the acetonitrile concentration was controlled to increase from 0 to 90%(v/v) in a linear gradient manner, and the liquid eluted from the column was fractionated by a prescribed volume. The eluted fractions were examined for IL-18-binding ability by the above-described binding assay. In fractions eluted at about 70%(v/v) acetonitrile, IL-18-binding ability was observed, and these fractions were pooled and concentrated. Thus a purified preparation of the IL-18-binding protein of human origin was obtained in a yield of about 3 μg.

This purified preparation of the IL-18-binding protein was examined for molecular weight by SDS-PAGE in the presence of dithiothreitol. A homogenous protein band bearing IL-18-binding ability was observed at the position of about 40,000 to 60,000 daltons. In addition, the IL-18-binding protein according to this Example was elucidated to be a glycoprotein by the fact that it adsorbed on "Wheat Germ Lectin Sepharose 6MB" of which ligand is wheat germ lectin.

Example 1-2: N-terminal amino acid sequence

A purified preparation of the IL-18-binding protein, obtained by the method in Example 1-1, was dried up by a centrifugal concentrator, treated with 0.1 M Tris-HCl buffer (pH 8.1) containing 8 M urea and 10 mM EDTA under a current of nitrogen gas at 50°C for 30 minutes, and reduced by an appropriate amount of dithiothreitol admixed therewith under a current of nitrogen gas at 50°C for 2 hours. This reaction mixture was admixed with an appropriate amount of monoiodoacetic acid and reacted under dark conditions at ambient temperature for 30 minutes to alkylate the IL-18-binding protein.

The above-obtained, alkylated product was subjected to SDS-PAGE in the presence of dithiothreitol. A protein corresponding to a molecular weight of about 40,000 to about 60,000 daltons was separated, and transferred to a PDVF membrane. The membrane was subjected to amino acid analysis with protein sequencer ("Type 473A," produced by Applied Biosystems) to determine the N-terminal amino acid sequence. The IL-18-binding protein of this invention according to Example 1-1 was proved to comprise the amino acid sequence shown in SEQ ID NO:3 ("Xaa" means an unidentified amino acid.) as the N-terminal amino acid sequence.

Example 1-3: Peptide mapping

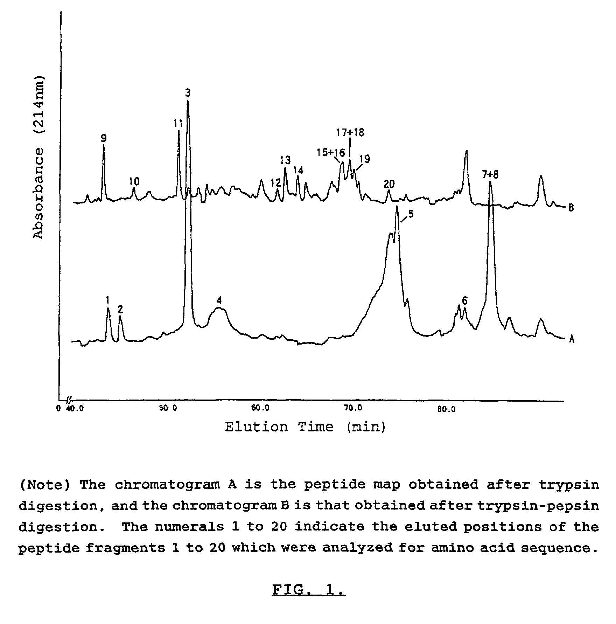

By the method "in-gel digestion" described in Ulf Hellman et al., *"Analytical Biochemistry*," Vol.224, pp.451-455 (1995), peptide maps of the IL-18-binding protein were prepared from the IL-18-binding protein which was reduced and alkylated by the method in Example 1-2 and then digested with trypsin or trypsin-pepsin. Further, the trypsin-produced peptide fragments 1 to 8 and trypsin-pepsin-produced peptide fragments 9 to 20 were sequenced. The peptide fragments 1 to 20 were proved to have the amino acid sequences shown in SEQ ID NOs:4 to 23 ("Xaa" means an unidentified amino acid.), respectively. The above-prepared peptide maps are shown in FIG. 1.

Example 1-4: IL-18-suppressive activity

A test for IL-18-suppressive activity was conducted similarly as in Example 3-3, described below, except for using lymphocytes from a healthy human, recombinant human IL-18, and standard human IFN-γ (Gg02-901-530) obtained from National Institute of Health of U.S.A. as immunocompetent cells, IL-18, and IFN-γ standard, respectively.

The induction of IFN-γ production by the action of human IL-18 was significantly suppressed by the co-existence of the IL-18-binding protein according to Example 1. This indicates that this IL-18-binding protein suppresses the physiological activities of IL-18.

Example 2: DNA encoding IL-18-binding protein of human origin

Example 2-1: DNA encoding IL-18-binding protein of human origin

Example 2-1(a): Nucleotide sequence of DNA encoding IL-18-binding protein of human origin

Ten nanograms of human liver poly(A)$^+$ RNA (product of Clontech) was mixed with 2 μl of 10 x PCR buffer, 2 μl of 25 mM magnesium chloride, 2 μl of 0.1 M dithiothreitol, 1 μl of 25 mM dNTP mix, 1 μl of 200 units/μl reverse transcriptase ("Superscript II," produced by Life-Tech Oriental Co., Ltd.), and 1 μl of 2.5 μM random hexamer, and the total volume was adjusted to 20 μl with sterilized-distilled water. This mixture was placed in a 0.5 ml reaction tube, and incubated sequentially at 42°C for 50 minutes and 70°C for 15 minutes to effect reverse transcriptase reaction. Thus a reaction product containing first strand cDNA was obtained.

This reaction product was admixed with 2.5-fold volumes of ethanol and 2 μl of 3 M sodium acetate, and allowed to stand at -20°C for 2 hours to precipitate the cDNA. The precipitate was collected, washed with 75%(v/v) ethanol in water, dissolved in sterilized-distilled water, admixed with 0.5 μl of 2.5 units/μl DNA polymerase ("Cloned Pfu polymerase," product of Stratagene), 10 μl of its specific buffer, and 1 μl of 25 mM dNTP mix, and further admixed with the oligonucleotide shown by 5'-ACNCCNGTNWSNCA-3' as a sense primer, chemically synthesized on the basis of the amino acid sequence of SEQ ID NO:3, and the oligonucleotide shown by 5'-TGNGCNARNACNACRTG-3' as an antisense primer, chemically synthesized on the basis of the amino acid sequence of SEQ ID NO:8, both in a volume of 10 μM, and the total volume was adjusted to 100 μl with sterilized-distilled water. This mixture was incubated under 40 cycles of the sequential conditions at 94°C, 40°C, and 72°C for 1 minute each to effect PCR.

A portion of the PCR product was collected and then electrophoresed on 1%(w/v) agarose gel to separate DNA fragments, and the DNA fragments were transferred to a nylon membrane and fixed thereon with 0.4 N sodium hydroxide. The membrane was washed with 2 x SSC, dried in air, immersed in prehybridization solution containing 6 x SSPE, 5 x Denhardt's solution, 0.5%(w/v) SDS, and 100 μg/ml denatured salmon sperm DNA, and incubated at 65°C for 3 hours. A probe was prepared by chemical synthesis of the oligonucleotide shown by 5'-GGRCANGGRTCYTT-3', based on the amino acid sequence shown in SEQ ID NO:3, and isotope-labeling thereof with [γ-$^{32}$P]ATP by T4 polynucleotide kinase. To the pre-hybridization solution in which the above nylon membrane had been immersed, 1 pmol of the probe was added, and the nylon membrane was incubated at 40°C for another 20 hours to effect hybridization. The nylon membrane was washed with 6 x SSC and subjected to autoradiography in a usual manner. A specific hybridization signal by the probe was observed. This showed that the above PCR product contained the objective DNA fragment.

To the remaining part of the above PCR product, 1 ng of a plasmid vector ("pCR-Script Cam SK(+)," produced by Stratagene) was added, and the DNA fragment of the PCR product was inserted into the vector with a DNA ligation kit ("DNA Ligation Kit, Version 2," produced by Takara Shuzo Co., Ltd.). With a portion of the reaction mixture collected, an *Escherichia coli* strain ("XL1-Blue MRF' Kan," produced by Stratagene) was transformed. The transformant was inoculated in LB medium (pH 7.5) containing 30 μg/ml chloramphenicol and cultured at 37°C for 18 hours. The cells were collected from the culture. The plasmid DNA was collected from the cells in a usual manner, and analyzed by dideoxy method. This plasmid DNA comprised the nucleotide sequence shown in SEQ ID NO:34 as the sequence of the DNA fragment produced by PCR. The amino acid sequence encoded by this nucleotide sequence, aligned therewith, were compared with the partial amino acid sequences determined in Examples 1-2 to 1-3, shown in SEQ ID NOs: 3 to 23. These partial amino acid sequences were completely or partly included by the amino acid sequence aligned in SEQ ID NO:34. This suggested that the nucleotide sequence shown in SEQ ID NO:34 encodes at least a part of the IL-18-binding protein of human origin.

Example 2-1(b): Nucleotide sequence encoding IL-18-binding protein of human origin

Ten nanograms of human liver poly(A)$^+$ RNA (product of Clontech) was subjected to 5'RACE, a modified method of PCR, with a commercially available 5'RACE kit ("5'RACE System, Version 2.0," product of GIBCO BRL). First, reverse transcriptase reaction was effected on the above RNA with the oligonucleotide shown by 5'-GGTCACTTCCAATGCTGGACA-3' as a primer, chemically synthesized on the basis of the nucleotide sequence shown in SEQ ID NO:34, and to the 5'-terminal of the first strand cDNA synthesized thereby, C-tail was added by the action of terminal deoxynucleotidyl transferase. Then, PCR was effected on this first strand cDNA with the oligonucleotide shown by 5'-GGCCACGCGTCGACTAGTACGGGIIGGGIIGGGIIG-3' as a sense primer, included by the above kit, and the oligonucleotide shown by 5'-GTCCTTTGTGCTTCTAACTGA-3' as an antisense primer, chemically synthesized on the basis of the nucleotide sequence shown in SEQ ID NO:34. A portion of the product of this 5'RACE was collected, and electrophoresed in a usual manner on 1%(w/v) agarose. Specific amplification of a DNA fragment was observed. This DNA fragment was sequenced similarly as in Example 2-1(a). This fragment comprised the nucleotide sequence shown in SEQ ID NO:35. The sequence from the 160th to 216th nucleotides of this sequence completely matched with the sequence from the 1st to 57th nucleotides of the nucleotide sequence shown in SEQ ID NO:34, determined in Example 2-1(a). This suggested that the nucleotide sequence shown in SEQ ID NO:35 overlaps with the nucleotide sequence shown in SEQ ID NO:34, encoding at least a part of the IL-18-binding protein of human origin, and comprises the 5'-upstream region of SEQ ID NO:34.

Example 2-1(c): Nucleotide sequence encoding IL-18-binding protein of human origin

Ten nanograms of human liver poly(A)$^+$ RNA was subjected to 3'RACE, a modified method of PCR, in accordance with "*PCR Jikken Manual* (Manual for PCR Experiments)," translated by Takashi Saito, published by HBJ Press (1991), 25-33. First, reverse transcriptase reaction was effected on the above RNA with the oligonucleotide shown by 5'-GACTCGAGTCGACATCGA(T)$_{17}$-3' as a primer. Then, PCR was effected on the first strand cDNA synthesized thereby with the oligonucleotide shown by 5'-TTCTCCTGTGTGCTCGTGGA-3' as a sense primer, chemically synthesized on the basis of the nucleotide sequence shown in SEQ ID NO:34, determined in Example 2-1(a), and the oligonucleotide shown by 5'-GACTCGAGTCGACATCG-3' as an antisense primer. A portion of the product of this 3'RACE was collected and electrophoresed in a usual manner on 1%(w/v) agarose. Specific amplification of a DNA fragment was observed. This DNA fragment was sequenced similarly as in Example 2-1(a). This fragment comprised the nucleotide sequence shown in SEQ ID NO:36. The sequence from the 1st to 60th nucleotides of this sequence completely matched with the sequence from the 352nd to 411st nucleotides of the nucleotide sequence shown in SEQ ID NO:34, determined in Example 2-1(a). This suggested that the nucleotide sequence shown in SEQ ID NO:36 overlaps with the nucleotide sequence shown in SEQ ID NO:34, encoding at least a part of the IL-18-binding protein of human origin, and comprises the 3'-downstream region of SEQ ID NO:34.

As described above, in Examples 2-1(a) to 2-1(c), the nucleotide sequences shown in SEQ ID NOs:34 to 36 were determined as ones partially encoding the IL-18-binding protein of human origin and overlapping one another. In view of the overlapping sequences, these three nucleotide sequences would be derived from one contiguous nucleotide sequence, which is shown in SEQ ID NO:37.

Example 2-1(d): Nucleotide sequence of DNA encoding human-derived IL-18-binding protein

In accordance with the method in Example 2-1(a), reverse transcriptase reaction was effected on human liver poly(A)$^+$ RNA, and then PCR was effected similarly as in Example 2-1(a) except for using as a sense primer the oligonucleotide shown by 5'-TGTGTGACTGGAGAAGAGGAC-3', chemically synthesized on the basis of the nucleotide sequence shown in SEQ ID NO:37, and as an antisense primer the oligonucleotide shown by 5'-TACAGGCAGTCAGGGACTGTTCACTCCAG-3', chemically synthesized on the basis of the nucleotide sequence shown in SEQ ID NO: 37. A portion of the PCR product was collected, and electrophoresed in a usual manner on 1%(w/v) agarose gel. Specific amplification of a DNA fragment was observed. This DNA fragment was sequenced similarly as in Example 2-1(a). This fragment comprised the nucleotide sequence shown in SEQ ID NO:37. This supported that the nucleotide sequences shown in SEQ ID NOs:34 to 36, determined in Examples 2-1(a) to 2-1(c), are partial sequences of the contiguous nucleotide sequence shown in SEQ ID NO:37.

The amino acid sequence encoded by the nucleotide sequence shown in SEQ ID NO:37, aligned therewith, are compared with the partial amino acid sequences shown in SEQ ID NOs:4 to 23, determined in Example 1-3. These partial sequences were all included by the amino acid sequence aligned in SEQ ID NO:37 in the region from the 1st to 164th amino acids. In addition, the N-terminal amino acid sequence determined in Example 1-2, shown in SEQ ID NO:3, well matched with the amino acid sequence aligned in SEQ ID NO:37 in the region from the 1st to 22nd amino acids. These facts suggested that the nucleotide sequence shown in SEQ ID NO:37 can encode the IL-18-binding protein of human origin by the region from the 160th to 651st nucleotides and that this IL-18-binding protein may has, as its whole sequence, the sequence from the 1st to 164th amino acids of the amino acid sequence aligned with this nucleotide sequence. Thus suggested amino acid sequence of the IL-18-binding protein of human origin and the nucleotide sequence encoding this are shown in SEQ ID NOs:1 and 32 separately.

Example 2-2: Production of IL-18-binding protein of human origin by transformant

Example 2-2(a): Preparation of recombinant DNA

A DNA capable of encoding the IL-18-binding protein of human origin, obtained by the method in Example 2-1(d), was placed in a 0.5-ml reaction tube in an amount of 1 ng, and to this tube, 10 μl of 10 x PCR buffer, 1 μl of 25 mM dNTP mix, and 2.5 units/μl DNA polymerase ("Cloned Pfu polymerase," produced by Stratagene) were added. Appropriate amounts of the oligonucleotide shown by 5'-CTCGAGGCCACCATGACCATGAGACACAAC-3' as a sense primer, chemically synthesized on the basis of the nucleotide sequence shown in SEQ ID NO:32, and the oligonucleotide shown by 5'-GCGGCCGCTCATTAGTGATGGTGATGGTGATGACCCTGCTGCTGTGGACT-3' as an antisense primer, chemically synthesized on the basis of the nucleotide sequence shown in SEQ ID NO:32, were further added to the above tube, and the total volume was adjusted to 100 μl with sterilized-distilled water. PCR was effected by incubating this mixture under 3 cycles of the sequential conditions at 94°C for 1 minute, at 42°C for 2 minutes, and at 72°C for 3 minutes and then 35 cycles of the sequential conditions at 94°C for 1 minute, at 60°C for 2 minutes, and 72°C for 3 minutes. The PCR product was analyzed and manipulated similarly as in Example 2-1(a); the PCR product was confirmed to contain the objective DNA fragment, and a plasmid vector inserted with this DNA fragment was obtained. This plasmid DNA comprised the nucleotide sequence shown in SEQ ID NO:32, confirmed by sequencing similarly as in Example 2-1(a).

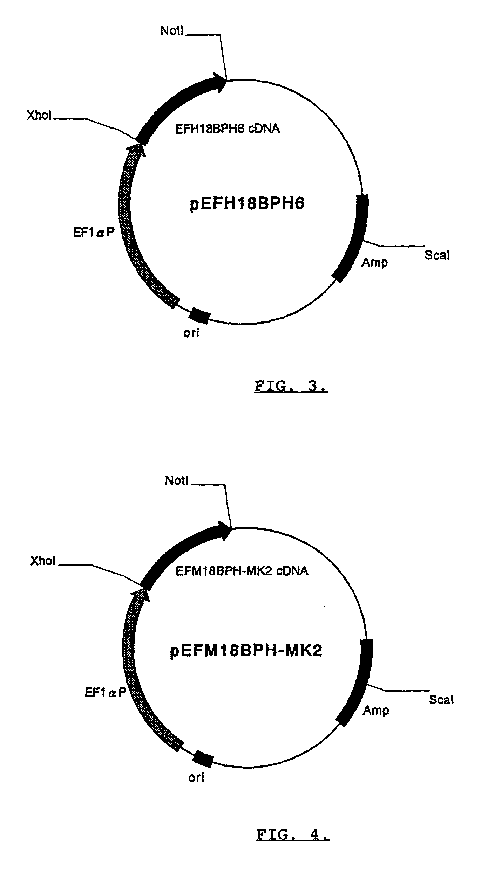

The restriction enzymes XAol and NotI were allowed to react in a usual manner on the above plasmid DNA to produce a DNA fragment. This DNA fragment was mixed with the plasmid vector "pEF-BOS", prepared similarly as in S. Mizushima et al., *"Nucleic Acid Research,"* Vol.17, No.18, p.5332 (1990) and digested with *Xho*I and *Not*I, at their proportion of 100 ng to 10 ng, and the DNA fragment was inserted into the plasmid vector with a DNA ligation kit ("DNA Ligation Kit, Version 2," produced by Takara Shuzo Co., Ltd.). Similarly as in Example 2-1(a), the *Escherichia coli* strain was transformed with this ligation product. From the resultant transformant, the recombinant DNA was collected, and named "pEFH18BPH6." This recombinant DNA was analyzed in a usual manner. As shown in FIG. 3, in the recombinant DNA "pEFH18BPH6," the cDNA "EFH18BPH6 cDNA" comprising the nucleotide sequence shown in SEQ ID NO:32, capable of encoding the IL-18-binding protein of human origin, was located on the downstream of the elongation factor 1 promotor "EF1αP."

Example 2-2(b): Production of IL-18-binding protein of human origin by transformant

The *Escherichia coli* strain transformed with the recombinant DNA "pEFH18BPH6" in Example 2-2(a) was inoculated in LB broth (pH 7.2) containing 100 μg/ml ampicillin, and cultured at 37°C under aerobic conditions by agitation. From the resultant culture, the plasmid DNA was collected in a usual manner to obtain the recombinant DNA "pEFH18BPH6". Twenty micrograms of this recombinant DNA was introduced by electroporation into 1 x $10^7$ cells of COS-1 (ATCC CRL-1650), a fibroblastic cell line derived from African green monkey kidney, which had been proliferated in a usual manner. Thus a transformant introduced with the DNA of this invention was obtained.

A medium ("ASF104," product of Ajinomoto) was placed in flat-bottomed culture flasks. The above-obtained transformant was inoculated into the medium at a ratio of 1 x $10^5$ cells/ml, and cultured in a 5% $CO_2$ incubator at 37°C for 3 days. The culture supernatant was collected from the resultant culture, and applied to a column with affinity chromatography gel ("Ni-NTA," product of QIAGEN). PBS containing 20 mM imidazole was fed to the column to remove non-adsorbed fraction, and then PBS containing 250 mM imidazole was fed while the liquid eluted from the column was fractionated by a prescribed volume. These fractions were examined for IL-18-binding ability by the above-described binding assay. Fractions with IL-18-binding ability were pooled. Thus an aqueous solution of purified IL-18-binding protein was obtained in a volume of about 2 ml. This solution contained about 10 μg/ml protein. After this solution was treated similarly as in Example 1-2, the N-terminal amino acid sequence was analyzed. The elucidated sequence was identical with the amino acid sequence shown in SEQ ID NO:3. As a control, procedures similar to this Example were conducted by using the plasmid vector "pEF-BOS" in place of the recombinant DNA "pEFH18BPH6." No IL-18-binding protein was observed. These results supported that the IL-18-binding protein of human origin usually has the amino acid sequence shown in SEQ ID NO:1 and can be encoded by the nucleotide sequence shown in SEQ ID NO:32.

Example 3: IL-18-binding protein of mouse origin

Example 3-1: Preparation of IL-18-binding protein

*Corynebacterium parvum* (ATCC 11827) was heated at 60°C for 1 hour. The dead cells thus obtained were injected with needles into 600 heads of 8-week-old, female CD-1 mice at a dose of 1 mg/head through intraperitoneal routes. The mice were housed in a usual manner for 7 days, and then injected with purified *Escherlchia coli* lipopolysaccharide through intravenous routes at a dose of 1 μg/head. Two hours later, the blood was collected from the mice's hearts, and by usual manipulation, 200 ml of serum was obtained from the blood. The serum was subjected to purification by the method in Example 1-1. Thus a purified preparation of the IL-18-binding protein of mouse origin was obtained in a yield of about 3 μg.

This purified preparation was examined for molecular weight by SDS-PAGE in the presence of dithiothreitol. A homogenous protein band bearing IL-18-binding ability was observed at the position of about 40,000 to 60,000 daltons. In addition, the IL-18-binding protein according to this Example was elucidated to be a glycoprotein by the fact that it adsorbed on "Wheat Germ Lectin Sepharose 6MB" of which ligand is wheat germ lectin.

Example 3-2: Peptide mapping

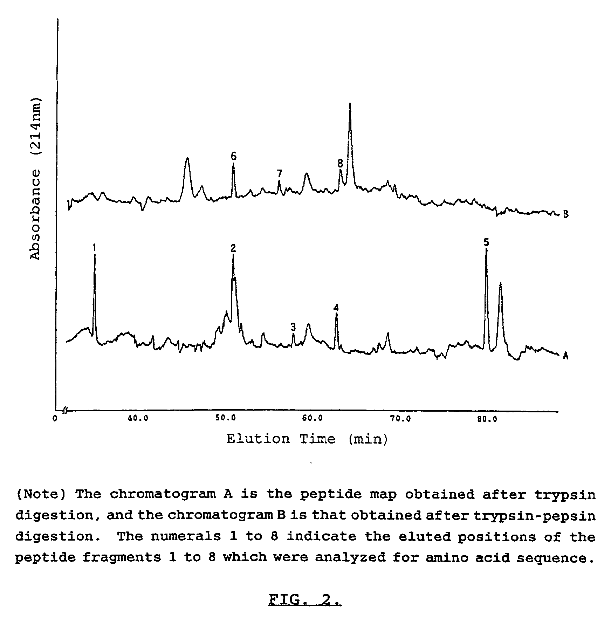

Similarly as in Example 1-3, peptide maps were prepared from a purified preparation of the IL-18-binding protein, obtained by the method in Example 3-1, and amino acid sequences were analyzed on the trypsin-produced peptide fragments 1 to 5 and trypsin-pepsin-produced peptide fragments 6 to 8. The peptide fragments 1 to 8 were proved to have the amino acid sequences shown in SEQ ID NOs:24 to 31 ("Xaa" means an unidentified amino acid.), respectively. The above-prepared peptide maps are shown in FIG. 2.

Example 3-3: IL-18-suppressive activity

Spleens were extracted from 14-week-old, female C3H/HeJ mice, and dispersed. After the adherent cells were removed, the spleen cells were suspended to use as immunocompetent cells in RPMI-1640 medium (pH 7.4) supplemented with 10%(v/v) fetal calf serum. The spleen cell suspension and 2.5 µg/ml concanavalin A were distributed to microplates at 0.15 ml and 0.05 ml per well. To each well, the above medium containing 25 ng/ml recombinant mouse IL-18 and a purified preparation of the IL-18-binding protein, prepared by the method in Example 3-1, at a content excessive to the IL-18, was added in a volume of 0.05 ml/well. The microplates were incubated in a 5% $CO_2$ incubator at 37°C for 24 hours. After the culture, 0.1 ml portion of each culture supernatant was collected, and measured for IFN-γ production by conventional enzyme-immunoassay. As controls, systems with no IL-18-binding protein or no mouse IL-18 were treated similarly as above. The measured values of IFN-γ were converted into international units (IU) with reference to the standard mouse IFN-γ (Gg02-901-533) obtained from National Institute of Health, U.S.A., as an IFN-γ standard.

IFN-γ produced in the control with no IL-18-binding protein was about 600 IU/ml, and that in the other control, with no mouse IL-18, was 0 IU/ml. In the test system with IL-18-binding protein, IFN-γ was produced only about 60 IU/ml. These results indicated that the IL-18-binding protein according to Example 3 suppresses the physiological activities of IL-18.

Example 4: DNA encoding IL-18-binding protein of mouse origin

Example 4-1: DNA encoding IL-18-binding protein of mouse origin

Example 4-1(a): Nucleotide sequence of DNA encoding IL-18-binding protein of mouse origin

*Corynebacterium parvum* (ATCC 11827) was heated at 60°C for 1 hour. The dead cells thus obtained were injected with needles into 8-week-old, female CD-1 mice at a dose of 1 mg/head through intraperitoneal routes. The mice were housed in a usual manner for 7 days, and then injected with purified *Escherichia coli* lipopolysaccharide through intravenous routes at a dose of 1 µg/head. Two hours later, the mice were slaughtered by dislocating each tibia, and the livers were extracted. Three grams by wet weight of the livers were immersed in 20 ml of a liquid (pH 7.0) consisting of 6 M guanidine isothiocyanato, 10 mM sodium citrate, and 0.5%(w/v) SDS, and disrupted with a homogenizer. In 35-ml centrifugal tubes, 0.1 M EDTA (pH 7.5) containing 5.7 M cesium chloride was poured in a volume of 25 ml/tube, and the cell disruptant was overlaid thereon at 10 ml/tube and then ultracentrifuged at 25,000 rpm for 20 hours at 20°C. The RNA fraction was collected, placed in a 15-ml centrifugal tube, and admixed with an equal volume of chloroform-isobutanol (4:1 by volume). The mixture was shaken for 5 minutes and centrifuged at 10,000 rpm for 10 minutes at 4°C, and the resultant liquid layer was collected. The liquid layer was admixed with 2.5-fold volumes of ethanol and allowed to stand at -20°C for 2 hours to precipitate total RNA. The precipitate was collected, washed with 75%(v/v) ethanol in water, and dissolved in 0.5 ml of sterilized-distilled water.

Reverse transcriptase reaction was effected similarly as in Example 2-1(a) on this total RNA, and PCR was effected on this reaction product containing first strand cDNA similarly as in Example 2-1(a) except for using as a sense primer the oligonucleotide shown by 5'-GCNGTNCCNACNAA-3', chemically synthesized on the basis of the amino acid sequence shown in SEQ ID NO:27, and as an antisense primer the oligonucleotide shown by 5'-GTYTTNARNCCRTC-3', chemically synthesized on the basis of the amino acid sequence shown in SEQ ID NO:30. A probe was prepared from the oligonucleotide shown by 5'-SWNGTRTGNCCYTCYTT-3', chemically synthesized on the basis of the amino acid sequence shown in SEQ ID NO:24. By using this probe and by the procedure according to Example 2, the above PCR product was confirmed to contain the objective DNA fragment. This DNA fragment was sequenced similarly as in Example 2-1(a). This fragment comprised the nucleotide sequence shown in SEQ ID NO:38. The amino acid sequence aligned in SEQ ID NO:38 was compared with the partial amino acid sequences shown in SEQ ID NOs: 24 to 31, determined in Example 3-2. These partial amino acid sequences were completely or partly included by the amino acid sequence aligned in SEQ ID NO:38. This suggested that the nucleotide sequence shown in SEQ ID NO: 38 encodes at least a part of the IL-18-binding protein of mouse origin.

Example 4-1(b): Nucleotide sequence of DNA encoding IL-18-binding protein of mouse origin

Total RNA was collected similarly as in Example 4-1(a) from female CD-1 mice treated with the dead cells of *Corynebacterium parvum* and lipopolysaccharide, and 1 µg of the total RNA was subjected to 5'RACE, a modified method of PCR, with a commercially available 5'RACE kit ("5'RACE System, Version 2.0," product of GIBCO BRL). First, reverse transcriptase reaction was effected on the above total RNA with the oligonucleotide shown by 5'-TGCAGGCAGTACAGGACAAGG-3' as a primer, chemically synthesized on the basis of the nucleotide sequence shown in SEQ ID NO:38, and to the 5'-terminal of the first strand cDNA synthesized thereby, C-tail was added by the action of terminal deoxynucleotidyl transferase. Then, PCR was effected on this first strand cDNA with the oligonucleotide shown by 5'-GGCCACGCGTCGACTAGTACGGGIIGGGIIGGGIIG-3' as a sense primer, included by the kit, and the oligonucleotide shown by 5'-GTGCTGGGTACTGCTTAGTTG-3' as an antisense primer. A portion of this 5'RACE product was collected, and electrophoresed in a usual manner on 1%(w/v) agarose gel. Specific amplification of a DNA fragment was observed. This DNA fragment was sequenced similarly as in Example 2-1(a). This fragment comprised the nucleotide sequence shown in SEQ ID NO:39. The sequence from the 307th to 336th nucleotides of this sequence completely matched with the sequence of the 1st to 30th nucleotides of the sequence shown in SEQ ID NO:38, determined in Example 4-1(a). This suggested that the nucleotide sequence shown in SEQ ID NO:39 overlaps with the nucleotide sequence shown in SEQ ID NO:38, encoding at least a part of the IL-18-binding protein of mouse origin, and comprises the 5'-upstream region of SEQ ID NO:38.

Example 4-1(c): Nucleotide sequence of DNA encoding IL-18-binding protein of mouse origin

Total RNA was collected similarly as in Example 4-1(a) from female CD-1 mice treated with the dead cells of *Corynebacterium parvum* and lipopolysaccharide, and 1 µg of the total RNA was subjected to 3'RACE, a modified method of PCR, in accordance with the methods described in "*PCR Jikken Manual* (Manual for PCR Experiments)," translated by Takashi Saito, published by HBJ Press (1991), pp.25-33. First, reverse transcriptase reaction was effected on the above total RNA with the oligonucleotide shown by 5'-GACTCGAGTCGACATCGA(T)$_{17}$-3' as a primer. Then, PCR was effected on the first strand cDNA synthesized thereby with the oligonucleotide shown by 5'-GATCCTGGACAAGTGGCC-3' as a sense primer, chemically synthesized on the basis of the nucleotide sequence shown in SEQ ID NO:38, determined in Example 4-1(a), and the oligonucleotide shown by 5'-GACTCGAGTCGACATCG-3' as an antisense primer. A portion of this 3'RACE product was collected, and electrophoresed in a usual manner on 1% (w/v) agarose gel. Specific amplification of a DNA fragment was observed. This DNA fragment was sequenced similarly as in Example 2-1(a). This fragment comprised the nucleotide sequence shown in SEQ ID NO:40. The sequence from the 1st to 63rd nucleotides of this sequence completely matched with the sequence of the 289th to 351st nucleotides of the sequence shown in SEQ ID NO:38, determined in Example 4-1(a). This suggested that the nucleotide sequence shown in SEQ ID NO:40 overlaps with the nucleotide sequence shown in SEQ ID NO:38, encoding at least a part of the IL-18-binding protein of mouse origin, and comprises the 3'-downstream region of SEQ ID NO:38.

As described above, in Examples 4-1(a) to 4-1(c), the nucleotide sequences shown in SEQ ID NOs:38 to 40 were determined as ones partially encoding the IL-18-binding protein of mouse origin and overlapping one another. In view of the overlapping sequences, these three nucleotide sequences would be derived from one contiguous nucleotide sequence, which is shown in SEQ ID NO:41.

Example 4-1(d) : Nucleotide sequence of DNA encoding IL-18-binding protein of mouse origin

Total RNA was collected similarly as in Example 4-1(a) from female CD-1 mice treated with the dead cells of *Corynebacterium parvum* and lipopolysaccharide. After reverse transcriptase reaction was effected on this total RNA, PCR was effected similarly as in Example 4-1(c) except for using the oligonucleotide shown by 5'-CTGAGCCTTAGAGCTCCAAG-3' as a sense primer and the oligonucleotide shown by 5'-GTGAAGCTTGAGTTTGAGGTTC-3' as an antisense primer, both chemically synthesized on the basis of the nucleotide sequence shown in SEQ ID NO:41. A portion of this PCR product was collected, and electrophoresed in a usual manner on 1%(w/v) agarose gel. Specific amplification of a DNA fragment was observed. This DNA fragment was sequenced similarly as in Example 2-1(a). This fragment comprised the nucleotide sequence shown in SEQ ID NO:41. This supported that the nucleotide sequences shown in SEQ ID NOs:38 to 40, determined in Examples 4-1(a) to 4-1(c), are partial sequences of the contiguous nucleotide sequence shown in SEQ ID NO:41.

The amino acid sequence encoded by the nucleotide sequence shown in SEQ ID NO:41, aligned therewith, are compared with the partial amino acid sequences shown in SEQ ID NOs:24 to 31, determined in Example 3-2.

These partial sequences were all included by the amino acid sequence aligned in SEQ ID NO:41 in the region from the 1st to 165th amino acids. In addition, the amino acid sequence of the IL-18-binding protein of human origin shown in SEQ ID NO:1 exhibited about 61% homology with the amino acid sequence aligned in SEQ ID NO:41 in the region from the 1st to 165th amino acids. These facts suggested that the nucleotide sequence shown in SEQ ID NO:41 can encode the IL-18-binding protein of mouse origin by the region from the 235th to 729th nucleotides and that this IL-18-binding protein may have, as its whole sequence, the sequence from the first to 165th amino acids of the amino acid sequence aligned with this nucleotide sequence. The amino acid sequence thus suggested as that of the IL-18-binding protein of mouse origin and the nucleotide sequence encoding this are shown in SEQ ID NOs:2 and 33 separately.

Example 4-2: Production of IL-18-binding protein of mouse origin by transformant

Example 4-2(a): Preparation of recombinant DNA

A DNA capable of encoding the IL-18-binding protein of mouse origin, obtained by the method in Example 4-1(d), was placed in a 0.5-ml reaction tube in an amount of 1 ng, and this DNA was treated similarly as in Example 2-2(a) except for using the oligonucleotide shown by 5'-CTCGACGCCACCATGACCATGAGACACTGC-3' as a sense primer and the oligonucleotide shown by 5'-GCGGCCGCTCATTAGTGATGGTGATGGTGATGTGCAACCCCTGGGCCTGC-3' as an antisense primer, both on the basis of the nucleotide sequence shown in SEQ ID NO:33. Similarly as in Example 4-1(a), the PCR product was confirmed to contain the objective DNA fragment, and a plasmid vector inserted with this DNA fragment was obtained. This plasmid DNA was sequenced similarly as in Example 2-1(a). The plasmid DNA comprised the nucleotide sequence shown in SEQ ID NO:33.
DNA insertion was effected from the above-obtained plasmid DNA into the plasmid vector "pEF-BOS" similarly as in Example 2-2(a). Thus obtained recombinant DNA was named "pEFM18BPH-MK2." This recombinant DNA was analyzed in a usual manner. As shown FIG. 4., in the recombinant DNA "pEFM18BPH-MK2," the cDNA "EFM18BPH-MK2 cDNA" comprising the nucleotide sequence shown in SEQ ID NO:33, capable of encoding the IL-18-binding protein of mouse origin, was located on the downstream of the elongation factor 1 promotor "EFαP."

Example 4-2(b): Production of IL-18-binding protein of mouse origin by transformant

From the culture of the *Escherichia coil* strain transformed with the recombinant DNA "pEFM18BPH-MK2" in Example 4-2, the plasmid DNA was collected in a usual manner to obtain the recombinant DNA "pEFM18BPH-MK2." Twenty micrograms of this recombinant DNA was introduced into COS-1 cells (ATCC CRL-1650) similarly as in Example 2-2(b). Thus a transformant introduced with the DNA of this invention was obtained.
Similarly as in Example 2-2(b), the above transformant was cultured, and the culture supernatant was collected and fractionated through a column with affinity chromatography gel ("Ni-NTA," product of QIAGEN). Fractions in which IL-18-binding protein was observed were collected and pooled. Thus an aqueous solution of purified IL-18-binding protein was obtained in a volume of about 2 ml from $1 \times 10^7$ cells of the transformant. This solution contained about 1 μg/ml protein. After this solution was treated according to Example 1-2, the N-terminal amino acid sequence was analyzed. The elucidated sequence was identical with the amino acid sequence shown in SEQ ID NO:2. As a control, procedures similar to this Example were conducted by using the plasmid vector "pEF-BOS" in place of the recombinant DNA "pEFH18BPH6." No IL-18-binding protein was observed. These results supported that the IL-18-binding protein of mouse origin usually has the amino acid sequence shown in SEQ ID NO:2 and can be encoded by the nucleotide sequence shown in SEQ ID NO:33.
The following are to explain the agent for susceptive disease containing the IL-18-binding protein of this invention as an effective ingredient.

Example 5: Solution

A purified preparation of the IL-18-binding protein, obtained by the method in Example 1-1 or 2-2, was dissolved to give a concentration of 1 mg/ml in physiological saline containing as a stabilizer 1%(w/v) pulverized crystalline trehalose ("Trehaose," commercialized by Hayashibara Shoji, Inc.) free from pyrogen. These solutions were made germ free in a usual manner. Thus two types of solutions were obtained.
These products, having excellent stability, are useful as an injection, ophthalmic solution, collunarium, etc. to treat or prevent the susceptive diseases including autoimmune diseases, inflammatory diseases, and allergic diseases.

Example 6: Dried infection

A purified preparation of the IL-18-binding protein, obtained by the method in Example 1-1 or 2-2, was dissolved at a ratio of 100 mg to 100 ml in physiological saline containing as a stabilizer 1%(w/v) sucrose free from pyrogen. These solutions were made germ free in a usual manner, distributed by 1 ml into vials, and lyophilized, and the vials were sealed.

These products, having excellent stability, are useful as a dried injection to treat or prevent the susceptive diseases including autoimmune diseases, inflammatory diseases, and allergic diseases.

Example 7: Ointment

Carboxyvinyl polymer ("Hi-Bis Wako," produced by Wako Pure Chemical Co., Ltd.) and pulverized crystalline trehalose ("Trehaose," commercialized by Hayashibara Shoji, Inc.) free from pyrogen were dissolved in sterilized-distilled water to give the respective concentrations of 1.4%(w/w) and 2.0%(w/w). This solution was mixed to a homogeneity with a purified preparation of the IL-18-binding protein, obtained by the method in Example 1-1 or 2-2, and then adjusted to pH 7.2. Thus 2 types of paste containing about 1 mg/g IL-18-binding protein were obtained.

These products, having excellent spreadability and stability, are useful as an ointment to treat or prevent the susceptive diseases including autoimmune diseases, inflammatory diseases, and allergic diseases.

Example 8: Tablets

Pulverized anhydrous maltose ("Finetose," commercialized by Hayashibara Shoji, Inc.) free from pyrogen was mixed to homogeneity with a purified preparation of IL-18-binding protein, obtained by the method in Example 1-1 or 1-2, and Lumin as a cell activator. These mixtures were tableted in a usual manner so that two types of tablets, each piece (about 200 mg) containing about 1 mg of the IL-18-binding protein and about 1 mg of Lumin (produced by Nihon Kanko Shikiso Co., Ltd.), were obtained.

These products, having excellent ingestibility and stability as well as cell-activating activity, are useful as tablets to treat or prevent the susceptive diseases including autoimmune diseases, inflammatory diseases, and allergic diseases.

Experiment: Acute Toxicity Test

A purified preparations of the IL-18-binding protein, obtained by the method in Example 1-1, 2-2, 3-1, or 4-2 was administered orally, intraperitoneally, or intravenously to five-week-old ddy mice (body weight of 20 to 25 g) in a usual manner. These purified preparations of the IL-18-binding protein had LD50 of about 1 mg/mouse-body-weight or higher, through any administration route. This indicates that it is safe to incorporate the IL-18-binding protein of this invention into pharmaceuticals to be administered to humans and other mammals.

INDUSTRIAL APPLICABILITY

As described above, this invention is established on the basis of the finding of a novel protein which binds to IL-18. The protein of this invention suppresses the physiological activities of IL-18, which is responsible for activation of immune system, in humans and other mammals, and this protein exhibits a distinguished efficacy in alleviating rejection reactions associated with organ transplantation and in treating and preventing various diseases resulting from augmented immunoreactions.

## Claims

1. An interleukin-18-binding protein comprising a part or the whole of the amino acid sequence shown in SEQ ID NO: 1 or 2.

2. The interleukin-18-binding protein of claim 1, which comprises a part or the whole of the amino acid sequence shown in any one of SEQ ID NOs:3 to 31.

3. The interleukin-18-binding protein of claim 1 or 2, which exhibits a molecular weight of about 40,000 to about 60,000 daltons on SDS-polyacrylamide gel electrophoresis.

4. The interleukin-18-binding protein of claim 1, 2, or 3, which is obtainable from a mammalian body fluid.

5. A DNA encoding the interleukin-18-binding protein of any one of claims 1 to 4.

6. The DNA of claim 5, which comprises the nucleotide sequence shown SEQ ID NO:32 or 33, a nucleotide sequence homologous to said nucleotide sequence, or a nucleotide sequence complementary to said nucleotide sequence.

7. An interleukin-18-suppressor containing as an effective ingredient the interleukin-18-binding protein of any one of claims 1 to 4.

8. An agent for susceptive diseases containing as an effective ingredient the interleukin-18-binding protein of any one of claims 1 to 4.

9. The agent for susceptive diseases of claim 8 as an anti-immunopathic agent.

(Note) The chromatogram A is the peptide map obtained after trypsin digestion, and the chromatogram B is that obtained after trypsin-pepsin digestion. The numerals 1 to 20 indicate the eluted positions of the peptide fragments 1 to 20 which were analyzed for amino acid sequence.

FIG. 1.

(Note) The chromatogram A is the peptide map obtained after trypsin digestion, and the chromatogram B is that obtained after trypsin-pepsin digestion. The numerals 1 to 8 indicate the eluted positions of the peptide fragments 1 to 8 which were analyzed for amino acid sequence.

FIG. 2.

FIG. 3.

FIG. 4.

## INTERNATIONAL SEARCH REPORT

| International application No. |
|---|
| PCT/JP98/05186 |

**A. CLASSIFICATION OF SUBJECT MATTER**
Int.Cl$^6$ C07K14/54, C12P21/02, C12N15/24, A61K38/20

According to International Patent Classification (IPC) or to both national classification and IPC

**B. FIELDS SEARCHED**

Minimum documentation searched (classification system followed by classification symbols)
Int.Cl$^6$ C07K14/54, C12P21/02, C12N15/24, A61K38/20

Documentation searched other than minimum documentation to the extent that such documents are included in the fields searched

Electronic data base consulted during the international search (name of data base and, where practicable, search terms used)
SwissProt/PIR/GeneSeq, Genbank/EMBL/DDBJ/GeneSeq, WPI (DIALOG), BIOSIS (DIALOG)

**C. DOCUMENTS CONSIDERED TO BE RELEVANT**

| Category* | Citation of document, with indication, where appropriate, of the relevant passages | Relevant to claim No. |
|---|---|---|
| A | Mark D. Adams et al., "Initial assessment of human gene diversity and expression patterns based upon 83 million nucleotides of cDNA sequence", Nature (1995) Vol. 377, No. 6547 suppl. p.3-174 | 1-9 |
| A | Ushio Shimpei et al, "Cloning of the cDNA for human IFN-gamma-inducing factor, expression in Escherichia coli, and studies on the biologic activities of the protein", Journal of Immunology (1996) Vol. 156, No. 11 p.4274-4279 | 1-9 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 February, 1999 (17. 02. 99) | 2 March, 1999 (02. 03. 99) |

| Name and mailing address of the ISA/ | Authorized officer |
|---|---|
| Japanese Patent Office | |
| Facsimile No. | Telephone No. |

Form PCT/ISA/210 (second sheet) (July 1992)